(19)

**Europäisches Patentamt**
**European Patent Office**
**Office européen des brevets**

(11) **EP 2 705 832 B1**

(12) **EUROPÄISCHE PATENTSCHRIFT**

(45) Veröffentlichungstag und Bekanntmachung des Hinweises auf die Patenterteilung:
**03.07.2019 Patentblatt 2019/27**

(21) Anmeldenummer: **13180351.2**

(22) Anmeldetag: **14.08.2013**

(51) Int Cl.:
*A61K 8/37* (2006.01)      *A61Q 15/00* (2006.01)
*A61Q 17/02* (2006.01)      *A61Q 17/04* (2006.01)
*A61Q 19/00* (2006.01)      *A61K 8/06* (2006.01)
*C07C 69/33* (2006.01)      *B01F 17/00* (2006.01)

(54) **Polyglycerinester mit besonderer Oligomerenverteilung des Polyglycerins**

Polyglycerol esters with special oligomer distribution of polyglycerol

Esters de polyglycérine dotés d'une répartition particulière des oligomères de la polyglycérine

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.09.2012 DE 102012215707**

(43) Veröffentlichungstag der Anmeldung:
**12.03.2014 Patentblatt 2014/11**

(73) Patentinhaber: **Evonik Degussa GmbH**
**45128 Essen (DE)**

(72) Erfinder:
• **Meyer, Jürgen**
  **45134 Essen (DE)**
• **Friedrich, Achim**
  **45529 Hattingen (DE)**
• **Fötsch, Hannelore**
  **45355 Essen (DE)**
• **Springer, Oliver**
  **46485 Wesel (DE)**
• **von Hof, Jan Marian**
  **45138 Essen (DE)**
• **Wenk, Hans Henning**
  **45470 Mülheim an der Ruhr (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 1 623 694      WO-A1-2008/103289**
**WO-A1-2011/098311      WO-A1-2012/127129**

Bemerkungen:
Die Akte enthält technische Angaben, die nach dem Eingang der Anmeldung eingereicht wurden und die nicht in dieser Patentschrift enthalten sind.

**Beschreibung**

Gebiet der Erfindung

Gegenstand der Erfindung sind neue Polyglycerinester mit besonderer Oligomerenverteilung des Polyglycerins sowie deren Verwendung in insbesondere kosmetischen Formulierungen, vorzugsweise als Emulgator.

Stand der Technik

In den letzten Jahren ist auf dem Kosmetikmarkt ein starker Trend zu Produkten auf Basis nachwachsender Rohstoffe zu verfolgen. Um diesen erfüllen zu können, ist es notwendig, leistungsstarke Emulgatoren auf Basis nachwachsender Rohstoffe anbieten zu können. Gängige Emulgatoren in der Kosmetik enthalten als hydrophile Gruppen oft Polyethylenglykolgruppen (PEG), die durch Polymerisation von petrochemisch gewonnenem Ethylenoxid hergestellt werden können. Da in möglichst natürlichen Formulierungen alle eingesetzten Rohstoffe aus nachwachsenden Quellen stammen sollten, sind PEG-haltige Emulgatoren in solchen Formulierungen nicht erwünscht.

Polyglycerinester sind ein bevorzugte PEG-freie Alternative für kosmetische Emulgatoren auf Basis nachwachsender Rohstoffe.

Der Einsatz von Polyglycerinestern in Kosmetika als Emulgator ist eine altbekannte Technik.

So beschreibt die JP 2003104852 die Verwendung eines bei Raumtemperatur festen Polyglycerin-Behenat-Stearats in Haarpflegeprodukten.

Die JP 2010178723 beschreibt die Verwendung von Polyglycerinpartialestern von Behensäure in Verbindung mit anderen, kürzerkettigen Fettsäuren als Teig-Verbesserer in Backwaren.

JP 2006055029 beschreibt die Verwendung von Triglycerin-Monobehenate-Monostearat in Pflanzenschutzanwendungen.

Des weiteren sind Polyglycerylester aus den Druckschriften WO 2008103289, EP 1623694 und WO 2011098311 bekannt.

Mit den im Stand der Technik beschriebenen Polyglycerinestern ist es nicht möglich, stabile Emulsionen mit gutem Hautgefühl trotz niedriger Viskosität, insbesondere in Verbindung mit hohen Konzentrationen an emulsionsbelastenden Inhaltsstoffen, wie beispielsweise Elektrolyten, Wirkstoffen, UV-Filtern oder Konservierungsmitteln, herzustellen.

Aufgabe der Erfindung war es, einen Emulgator bereitzustellen, der mindestens einen Nachteil des Standes der Technik zu überwinden vermag.

Beschreibung der Erfindung

Überraschenderweise wurde gefunden, dass die im Folgenden beschriebenen Polyglycerinester die der Erfindung gestellt Aufgabe zu lösen vermögen.

Gegenstand der vorliegenden Erfindung sind daher Polyglycerinester wie in Anspruch 1 und davon abhängigen Ansprüchen beschrieben.

Ein weiterer Gegenstand der Erfindung ist ein Verfahren zur Herstellung der erfindungsgemäßen Polyglycerinester sowie deren Verwendung als Emulgator und Formulierungen, die diese enthalten.

Ein Vorteil der vorliegenden Erfindung besteht darin, dass der bereitgestellte Emulgator auf Basis des erfindungsgemäßen Polyglycerinesters vollkommen auf nachwachsenden Rohstoffen basiert.

Noch ein Vorteil der vorliegenden Erfindung ist es, dass der bereitgestellte Emulgator auf Basis des erfindungsgemäßen Polyglycerinesters sich zur Formulierung von O/W Emulsionen (Cremes, Lotionen) mit hervorragender Lagerstabilität eignet.

Ein weiterer Vorteil der vorliegenden Erfindung ist es, dass der bereitgestellte Emulgator auf Basis des erfindungsgemäßen Polyglycerinesters sich zur Formulierung PEG-freier Emulsionen, insbesondere dünnflüssiger PEG-freier Emulsionen eignet.

Emulsionen und Formulierungen enthaltend solchen Emulgator auf Basis des erfindungsgemäßen Polyglycerinesters weisen überdies ein gutes Hautgefühl auf. Der Emulgator auf Basis des erfindungsgemäßen Polyglycerinesters kann Emulsionen gegenüber emulsionsbelastenden Inhaltsstoffen stabilisieren. Hier sind beispielweise Sonnenschutzformulierungen enthaltend UV-Filter, elektrolythaltige Formulierungen und Formulierungen mit kosmetischen Wirkstoffen, sowie insbesondere pigmenthaltige Emulsionen genannt.

Vorteilhafterweise benötigen Emulsionen und Formulierungen enthaltend solchen Emulgator auf Basis des erfindungsgemäßen Polyglycerinesters keine parabenhaltigen Konservierungsmittel.

Ein weiterer Vorteil der vorliegenden Erfindung ist es, dass der bereitgestellte Emulgator sich zur Formulierung von Emulsionen ohne polyacrylat-basierte Verdicker eignet.

Ein weiterer Vorteil der vorliegenden Erfindung ist es, dass der bereitgestellte Emulgator aufgrund seiner Konsistenz gut handhabbar ist.

Ein weiterer Vorteil der vorliegenden Erfindung ist es, dass der bereitgestellte Emulgator in Formulierungen ein leichtes Hautgefühl erzeugt.

Ein weiterer Vorteil der vorliegenden Erfindung ist es, dass die Verwendung des bereitgestellten Emulgators den Formulierungen feuchtigkeitsspendende Eigenschaften verleiht.

Die Zusammensetzung aus natürlichen Rohstoffen sowie die Fähigkeit des Emulgators auf Basis des erfindungsgemäßen Polyglycerinesters, Emulsionen auch ohne polyacrylat-basierte Verdicker zu stabilisieren, ermöglicht die Herstellung natürlicher Emulsionen bspw. nach Natrue- oder Cosmoskriterien. Das leichte Hautgefühl sowie die feuchtigkeitsspenden Eigenschaften der erfindungsgemäßen Polyglycerinemulgatoren empfehlen die Verwendung dieser in Seren, Feuchtigkeitslotionen, Anti-Aging-Formulierungen oder Blemish Balm Cremes (getönte Wirkstoffformulierung).

Ein weiterer Vorteil der vorliegenden Erfindung ist es, dass die Verwendung des bereitgestellten Emulgators den Formulierungen eine gute Wirksamkeit bezüglich eingearbeiteter Insektrepellentien speziell in Kombination mit UV Filtern, insbesondere wasserlöslichen UV Filtern, verleiht.

Ein weiterer Vorteil der vorliegenden Erfindung ist es, dass die Verwendung des bereitgestellten Emulgators den Formulierungen eine gute Stabilität bezüglich eingearbeiteter Insektrepellentien speziell in Kombination mit UV Filtern, insbesondere wasserlöslichen UV Filtern, verleiht.

Beansprucht wird daher ein Polyglycerinester, der nach seiner vollständiger Hydrolyse im Mittel (Zahlenmittel) pro Mol Polyglycerinester von 0,51 bis 2,20 Mol, bevorzugt von 0,73 bis 1,85 Mol, besonders bevorzugt von 0,85 bis 1,50 Mol, mindestens einer Carbonsäure mit 8 bis 24 Kohlenstoffatomen freisetzt, dadurch gekennzeichnet, dass nach vollständiger Hydrolyse des Polyglycerinesters ein Polyglycerin erhalten wird, in dem das Massenverhältnis von Glycerin zu Diglycerin größer 1, bevorzugt größer 1,2, besonders bevorzugt größer 1,5 ist, und das einen mittleren Polymerisationsgrad von 3 bis 20, bevorzugt von 3,5 bis 15 besonders bevorzugt von 4,5 bis 10 aufweist.

Bevorzugte erfindungsgemäße Polyglycerinester sind dadurch gekennzeichnet, dass nach ihrer vollständigen Hydrolyse im Mittel (Zahlenmittel) pro Mol Polyglycerinester von 0,01 bis 0,20 Mol, bevorzugt von 0,03 bis 0,15 Mol, besonders bevorzugt von 0,05 bis 0,10 Mol, mindestens einer ersten Carbonsäure mit 20 bis 24 Kohlenstoffatomen und von 0,5 bis 2,0 Mol, bevorzugt von 0,7 bis 1,7 Mol, besonders bevorzugt von 0,8 bis 1,4 Mol, mindestens einer zweiten Carbonsäure mit 8 bis 18, bevorzugt mit 14 bis 18, besonders bevorzugt von 16 bis 18 Kohlenstoffatomen, freigesetzt werden. Unter dem Begriff "Polyglycerin" im Sinne der vorliegenden Erfindung ist ein Polyglycerin zu verstehen, welches Glycerin enthält. Somit ist zur Berechnung von Mengen, Massen und dergleichen der Glycerinanteil mit zu berücksichtigen. Polyglycerine im Sinne der vorliegenden Erfindung sind somit Mischungen von Glycerin mit mindestens einem Glycerin-Oligomer.

Analoges gilt für den Begriff "Polyglycerinester" im Zusammenhang mit der vorliegenden Erfindung.

Das angegebene Zahlenmittel der Säurereste bezieht sich bei mehr als einer der ersten und/oder zweiten Carbonsäure jeweils auf die akkumulierte Summe aller ersten und/oder zweiten Säurereste.

Alle angegebenen Prozent (%) sind, wenn nicht anders angegeben, Massenprozent. Das Massenverhältnis von Glycerin zu Diglycerin sowie die weitere Homologenverteilung des Polyglycerins lässt sich im Rahmen der vorliegenden Erfindung nach der GPC-Methode bestimmen; diese Methode beinhaltet die alkalische Hydrolyse des erfindungsgemäßen Polyglycerinesters, Trennung des Polyglycerins von den entstandenen Säuren und Analyse durch Gel-Permeations-Chromatographie.

Hierzu werden 0,6 g des erfindungsgemäßen Polyglycerinesters in 25 ml einer ethanolischen 0,5 M KOH Lösung unter Rückfluss für 4 Stunden gekocht. Dann wird der pH mit Schwefelsäure auf pH 2-3 eingestellt, und die entstandenen Carbonsäuren werden durch dreimalige Extraktion mit jeweils einem Volumen Petrolether abgetrennt. Die vereinigten Extrakte werden durch Evaporation auf ca. 10 ml eingeengt.

Geeignete Bestimmungsmethoden zur Ermittlung der Fettsäureverteilung sind insbesondere solche gemäß DGF C VI 11a, DGF C-VI 10 a und GAT - Ringtest 7/99.

Ein 0,5 ml Aliquot des wie oben beschriebenen erhaltenen Petroletherextraktes wird in einem Autosamplergefäß mit 0,5 ml MTBE und 1 ml Trimethylanilinium Hydroxide (0.2 M in Methanol) versetzt und mittels GC analysiert. Dieses wird in einem Gaschromatograph, welcher mit einem split/splitless Injektor, einer Kapillarsäule und einem Flammenionisationdetektor ausgestattet ist, unter folgenden Bedingungen durchgeführt:

| | |
|---|---|
| Injektor: | 290 °C, Split 30 ml |
| Injektionsvolumen: | 1 μl |
| Säule: | 30 m *0,32 mm HP1 0,25 μm |
| Trägergas: | Helium, head pressure 70 kPa |
| Temperaturprogramm: | 80 °C - 300 °C mit 8 °C/min, dann 20 Minuten bei 300 °C konditionieren. |

(fortgesetzt)

| Detektor: | FID bei 320 °C |
| | Wasserstoff 35 ml/min |
| | Luft 240 ml/min |
| | Make Up Gas 12 ml/min |

Die Carbonsäuren werden als ihre Methylester nach ihrer Kohlenstoffkettenlänge getrennt. Durch Auswertung der Peakflächen kann das Massenverhältnis dieser Carbonsäuremethylester untereinander und mittels ihrer jeweiligen Molekulargewichte daraus ihr Stoffmengenverhältnis bestimmt werden, welches dem Stoffmengenverhältnis der zugehörigen Carbonsäuren entspricht. Außerdem kann ein mittleres Molekulargewicht dieser Fettsäuremischung bestimmt werden:

| | | |
|---|---|---|
| $$a_i = \frac{A_i}{\sum_i A_i} \cdot 100$$ | mit | $a_i$ = normierter Massenanteil des Carbonsäuremethylesters $i$ am Gemisch aller Carbonsäuremethylester [%]. $A_i$ = Peakfläche des Carbonsäuremethylesters $i$ im GC-Chromatogramm [%]. |
| $$n_i = \frac{a_i}{(M_i + 14)}$$ | mit | $n_i$ = Stoffmenge [mol] des Carbonsäuremethylesters $i$ in 100 g Carbonsäuremethylestermischung; hieraus werden die Verhältnisse der einzelnen $n_i$ untereinander erhalten, die den Stoffmengenverhältnissen der zugehörigen Carbonsäuren im Polyglycerinester entsprechen; somit kann die Gesamtstoffmenge $n_s$ [mol] der Carbonsäuren in 1 g Polyglycerinester, wie sie aus der Verseifungszahl erhalten wird (s.u.) den Verhältnissen entsprechend in ihre Komponenten aufgeteilt werden. $M_i$ = Molekulargewicht der dem Methylester entsprechenden Carbonsäure $i$ [g/mol]. |
| $$\overline{M_s} = \sum_i \left( \frac{n_i}{\sum_i n_i} \cdot M_i \right)$$ | mit | $\overline{M_s}$ = mittleres Molekulargewicht der Carbonsäuremischung. $n_i$ = Stoffmenge [mol] des Carbonsäuremethylesters $i$ in 100 g Carbonsäuremethylestermischung. $M_i$ = Molekulargewicht der Carbonsäure $i$ [g/mol]. |

Geeignete Bestimmungsmethoden zur Ermittlung der Verseifungszahl sind insbesondere solche gemäß DGF C-V 3 und Ph.Eur. 2.5.6.

Mit Hilfe der Verseifungszahl lässt sich darüber hinaus zunächst die in 1 g Polyglycerinester enthaltene Gesamtstoffmenge an Carbonsäure(n) und anschließend (mittels des wie oben beschrieben per GC-Analyse bestimmten Stoffmengenverhältnisses und der Molekulargewichte der Carbonsäuren) die Gesamtmasse an Carbonsäure(n) erhalten. Daraus wiederum lässt sich durch Subtraktion die in 1 g Polglycerinester enthaltene Masse an Polyglycerin bestimmen:

| | | |
|---|---|---|
| $$\frac{VZ}{1000 \cdot 56{,}11} = n_s$$ | mit | VZ = Verseifungszahl [mg KOH/g Polyglycerinester]. $n_s$ = Gesamtstoffmenge [mol] der Carbonsäuren in 1 g Polyglycerinester. |
| $1 - (\overline{M_s} \cdot n_s - n_s \cdot 17) + n_s \cdot 1 = m_p$ | mit | $\overline{M_s}$ = mittleres Molekulargewicht der Carbonsäuremischung [g/mol]. $n_s$ = Gesamtstoffmenge [mol] der Carbonsäuren in 1 g Polyglycerinester. $m_p$ = Masse Polyglycerin in 1 g Polyglycerinester [g]. |

Mittels des Molekulargewichts des Polyglycerins lässt sich die Stoffmenge des Polyglycerins bestimmen:

| | | |
|---|---|---|
| $M_p = 74 \cdot N + 18$ | mit | $M_p$ = Molekulargewicht des Polyglycerins [g/mol] N = Polymerisationsgrad des Polyglycerins (zur Bestimmung des Polymerisationsgrads siehe weiter unten). |

(fortgesetzt)

| | mit | $n_p$ = Stoffmenge des Polyglycerins [mol] in 1 g Polyglycerinester |
|---|---|---|
| $$n_p = \frac{m_p}{M_p}$$ | | $m_p$ = Masse Polyglycerin in 1 g Polyglycerinester [g] |
| | | $M_p$ = Molekulargewicht des Polyglycerins [g/mol] |

Zusammen lassen sich aus diesen Werten die Molverhältnisse von Polyglycerin zu Carbonsäuren bestimmen.

Der mit Petrolether extrahierte Rückstand wird mit Bariumhydroxid auf pH 7 bis 8 eingestellt. Das ausgefallene Bariumsulfat wird durch Zentrifugation abgetrennt. Der Überstand wird abgenommen und der Rückstand wird dreimal mit 20 ml Ethanol extrahiert.

Die vereinigten Überstände werden für 30 min bei 80 °C und 50 mbar eingeengt und getrocknet.

Für die Analyse der Polyglycerinfraktion mittels GPC wird vom Rückstand eine Probenkonzentration von 1 g/l im Eluenten, $H_2O$ mit 0,05% $NaN_3$, hergestellt. Ein Aliquot wird unter den folgenden Bedingungen analysiert:

| Gerät: | Pumpe: Agilent 1200, isokratisch |
|---|---|
| | Autosampler Agilent 1100 |
| Detektor: | Agilent RI |
| Probenkonzentration: | 1.000 g/L |
| Injektionsvolumen: | 50.000 µL |
| Säule: | SUPREMA 30Å, 8 mm x 300 mm und Vorsäule |
| Flussrate: | 0.5 mL/min |
| Säulentemperatur: | 30 °C |
| Software for Analysis: | PSS WinGPC Unity Version 7.5.0 |

Unter diesen Bedingungen wird das Polyglycerin nach dem Polymerisierungsgrad getrennt. Da die Auflösung der verschiedenen Oligomere gering ist und die Bestimmung der Peakflächen der einzelnen Oligomere durch eine Lotrechte im Minimum zwischen den Peaks zu ungenau ist, wird der relative Gehalt der einzelnen Oligomere (vornehmlich Glycerin und Diglycerin) durch Auswertung der Peakhöhen bestimmt.

Sollte in einem betrachteten Polyglycerin keine nachweisbare Menge an Diglycerin, jedoch Glycerin enthalten sein, so entspricht dies einem Massenverhältnis von Glycerin zu Diglycerin größer 1,2.

Es ist erfindungsgemäß bevorzugt, dass das nach vollständiger Hydrolyse des erfindungsgemäßen Polyglycerinesters erhaltene Polyglycerin einen mittleren Polymerisationsgrad von 3 bis 20, bevorzugt von 3,5 bis 15, besonders bevorzugt von 4,5 bis 10, aufweist.

Der mittlere Polymerisationsgrad des Polyglycerins $N$ wird über dessen Hydroxylzahl (OHV, in mg KOH/g) gemäß folgender Formel berechnet:

$$N = \frac{(112200 - 18 \cdot OHV)}{(74 \cdot OHV - 56100)}$$

Geeignete Bestimmungsmethoden zur Ermittlung der Hydroxylzahl sind insbesondere solche gemäß DGF C-V 17 a (53), Ph. Eur. 2.5.3 Method A und DIN 53240.

Vorteilhafte Polyglycerinester gemäß der vorliegenden Erfindung sind dadurch gekennzeichnet, dass das molare Verhältnis der nach vollständiger Hydrolyse des Polyglycerinesters erhaltenen ersten Carbonsäuren zur zweiten Carbonsäure zwischen 1 zu 50 bis 1 zu 7, bevorzugt zwischen 1 zu 28 bis 1 zu 10, besonders bevorzugt zwischen 1 zu 19 und 1 zu 13, liegt.

Zur Bestimmung der Molverhältnisse lässt sich als Methode die oben beschriebene Methode einsetzen.

Ein erfindungsgemäßer Polyglycerinester ist dadurch gekennzeichnet, dass die erste Carbonsäure 20 bis 24 Kohlenstoffatome und die zweite Carbonsäure 8 bis 18 Kohlenstoffatome enthält.

Es ist erfindungsgemäß bevorzugt, dass die erste und zweite Carbonsäure des Polyglycerinesters ausgewählt ist aus Fettsäuren, solche sind insbesondere ausgewählt aus linearen, gesättigten, unsubstituierten Carbonsäuren.

Als Fettsäuren lassen sich sowohl für die erste als auch die zweite Carbonsäure Mischungen von Fettsäuren einsetzen, wobei es für die zweite Carbonsäure bevorzugt ist, dass es sich in diesem Zusammenhang um eine Mischung handelt.

Dies liegt in der Natur des Herstellungsverfahrens begründet, in dem bevorzugt technische Mischungen von Fettsäuren eingesetzt werden.

Ein erfindungsgemäß bevorzugter Polyglycerinester ist dadurch gekennzeichnet, dass er als erste Carbonsäure solche enthält ausgewählt aus Arachinsäure, Behensäure und Lignocerinsäure, besonders bevorzugt Behensäure. Zusätzlich kommen auch monoolefinische ungesättigte Carbonsäuren als erste Carbonsäure in Betracht, z.B. Eicosen- und Docosensäuren wie Gadoleinsäure, Icosensäure, Cetoleinsäure, Erucasäure, Brassidinsäure, Nervonsäure.

Bevorzugte zweite Carbonsäure sind Laurinsäure, Tridecansäure, Myristinsäure, Palmitinsäure, Margarinsäure, Stearinsäure, Isostearinsäure, sowie Mischungen hieraus. Natürlich vorkommende Mischungen sind beispielsweise die Kokosfettsäuren, die als Hauptbestandteil Laurinsäure, daneben noch gesättigte $C_{14}$-$C_{18}$-Fettsäuren und gegebenenfalls gesättigte $C_8$-$C_{10}$-Fettsäuren und ungesättigte Fettsäuren enthalten. Palmitinsäure und Stearinsäure sind in diesem Zusammenhang die besonders bevorzugte zweite Carbonsäure. Diese können als Mischung, die im Gewichtsverhältnis Stearinsäure zu Palmitinsäure von 100:0,01 zu 0,01:100 breit variiert werden können, vorliegen. Bevorzugt ist dabei ein C18:C16-Gewichtsverhältnis von 30:70 bis 95:5, besonders ein Verhältnis von 45:55 bis 90:10.

Zusätzlich kommen auch die monoolefinisch ungesättigte Carbonsäuren als zweite Carbonsäure, beispielsweise Hexadecensäuren, Octadecensäuren, wie Ölsäure (cis-9-Octadecensäure) oder Elaidinsäure (trans-9-Octadecensäure) wie auch der mehrfach ungesättigte Fettsäuren, beispielsweise Octadecadiensäuren und Octadecatriensäuren, wie Linolsäure und Linolensäure, sowie Mischungen hieraus, in Betracht.

Eine besonders bevorzugt Ausführungsform des erfindungsgemäßen Polyglycerinesters ist dadurch gekennzeichnet, dass die erste Carbonsäure Behensäure und die zweite Carbonsäure eine Mischung von Stearinsäure und Palmitinsäure ist, die bevorzugt ein Gewichtsverhältnis in einem Bereich von 0,8 zu 1 bis 1,2 zu 1 aufweist.

Es ist erfindungsgemäß bevorzugt, dass der erfindungsgemäße Polyglycerinester einen Schmelzpunkt von größer 35°C, bevorzugt größer 40°C, insbesondere größer 50°C aufweist. Der Schmelzpunkt des Polyglycerinesters lässt sich dabei beispielsweise durch Variation des Gehalts an langkettigen, gesättigten Fettsäuren beeinflussen (siehe beispielsweise Ullmanns Enzyklopädie der Technischen Chemie oder Römpp Lexikon Chemie jeweils zu Fettsäuren).

Die Polyglycerinester der vorliegenden Erfindung lassen sich durch klassische Veresterungsverfahren herstellen, bevorzugt mit dem im Folgenden beschriebenen erfindungsgemäßen Verfahren.

Ein weiterer Gegenstand der vorliegenden Erfindung ist ein Verfahren zur Herstellung eines Polyglycerinesters umfassend die Verfahrensschritte

    A) Bereitstellen eines Polyglycerins, in dem das Massenverhältnis von Glycerin zu Diglycerin größer 1, bevorzugt größer 1,2, besonders bevorzugt größer 1,5 ist,

    B) Verestern mindestens eines Teils des Polyglycerins mit mindestens einer Carbonsäure mit 8 bis 24 Kohlenstoffatomen,

wobei das molare Verhältnis der in Verfahrensschritt B) eingesetzten Carbonsäure zu in Verfahrensschritt A) eingesetztem Polyglycerin von 0,51 zu 1 bis 2,20 zu 1, bevorzugt von 0,73 zu 1 bis 1,85 zu 1, besonders bevorzugt von 0,85 zu 1 bis 1,5 zu 1 beträgt.

Bevorzugte erfindungsgemäße Verfahren sind dadurch gekennzeichnet, dass in Verfahrensschritt B) mindestens eine erste Carbonsäure mit 20 bis 24 Kohlenstoffatomen und mindestens eine zweite Carbonsäure mit 8 bis 18 Kohlenstoffatomen eingesetzt wird, wobei das molare Verhältnis der in Verfahrensschritt B) eingesetzten ersten Carbonsäure zu in Verfahrensschritt A) eingesetztem Polyglycerin von 0,01 zu 1 bis 0,20 zu 1, bevorzugt von 0,03 zu 1 bis 0,15 zu 1, besonders bevorzugt von 0,05 zu 1 bis 0,10 zu 1, und das molare Verhältnis der in Verfahrensschritt B) eingesetzten zweiten Carbonsäure zu in Verfahrensschritt A) eingesetztem Polyglycerin von 0,5 zu 1 bis 2,0 zu 1, bevorzugt von 0,7 zu 1 bis 1,7 zu 1, besonders bevorzugt von 0,8 zu 1 bis 1,4 zu 1, beträgt.

Das Polyglycerin für Verfahrensschritt A) kann bereitgestellt werden durch verschiedene konventionelle Methoden wie beispielsweise Polymerisation von Glycidol (z.B. basenkatalysiert), Polymerisation von Epichlorhydrin (beispielsweise in Gegenwart äquimolarer Mengen einer Base wie NaOH) oder Polykondensation von Glycerin. Erfindungsgemäß bevorzugt ist die Bereitstellung des Polyglycerins durch die Kondensation von Glycerin, insbesondere in Gegenwart katalytischer Mengen einer Base, insbesondere NaOH oder KOH. Geeignete Reaktionsbedingungen sind Temperaturen zwischen 220-260 °C und reduzierter Druck in einem Bereich zwischen 20 bis 800 mbar, insbesondere zwischen 50 und 500 mbar, wodurch eine erleichterte Wasserentfernung möglich ist. Entsprechende Verfahren lassen sich Standardlehrwerken der Chemie wie beispielsweise dem Römpp entnehmen.

Es kann vorteilhaft sein, ein konventionell erhaltenes Polyglycerin durch Beimischen von Glycerin auf das erfindungsgemäß benötigte Verhältnis von Glycerin zu Diglycerin zu bringen.

Es ist bevorzugt, wenn das in Verfahrensschritt A) bereitgestellte Polyglycerin einen mittleren Polymerisationsgrad von 3 bis 20, bevorzugt von 3,5 bis 15 besonders bevorzugt von 4,5 bis 10 aufweist.

In dem erfindungsgemäßen Verfahren werden die Verfahrensschritte B) unter dem Fachmann bekannten Bedingungen für Veresterungsreaktionen, gegebenenfalls in Anwesenheit eines Katalysators, durchgeführt. Insbesondere wird diese Veresterung unter Entfernung von Wasser aus dem Reaktionsgemisch durchgeführt.

Verfahrensschritt B) wird bevorzugt bei 180-260 °C, besonders bevorzugt 210-250°C durchgeführt.

Der Reaktionsverlauf kann beispielsweise über die Säurezahl des Produktes überwacht werden, so dass es in Verfahrensschritt B) bevorzugt ist, diesen so lange durchzuführen, bis die gewünschte Säurezahl erreicht wird.

Bevorzugt werden in Verfahrensschritt B) die erste und zweite Carbonsäure in einem molaren Verhältnis zwischen 1 zu 50 bis 1 zu 7, bevorzugt zwischen 1 zu 28 bis 1 zu 10, besonders bevorzugt zwischen 1 zu 19 und 1 zu 13 eingesetzt. Die in dem erfindungsgemäßen Verfahren in Verfahrensschritt B) eingesetzten ersten und zweiten Carbonsäuren sind bevorzugt solche, die oben als bevorzugte in erfindungsgemäßen Polyglycerinestern genannt wurden.

Insbesondere werden in diesem Zusammenhang als die erste Carbonsäure Behensäure und als die zweite Carbonsäure eine Mischung von Stearinsäure und Palmitinsäure, die bevorzugt ein Gewichtsverhältnis von Stearinsäure zu Palmitinsäure in einem Bereich von 0,8 zu 1 bis 1,2 zu 1 aufweist, eingesetzt. Bevorzugt werden in diesem Zusammenhang erste und zweite Carbonsäure in einem molaren Verhältnis 1 zu 50 bis 1 zu 7, bevorzugt zwischen 1 zu 28 bis 1 zu 10, besonders bevorzugt zwischen 1 zu 19 und 1 zu 13 eingesetzt.

Erfindungsgemäße Polyglycerinester und Polyglycerinester erhältlich bzw. erhalten nach dem erfindungsgemäßen Verfahren eignen sich hervorragend zur Verwendung als leistungsstarker O/W Emulgator, der ausschließlich auf nachwachsenden Rohstoffen basiert und eine große Formulierungsflexibilität aufweist, insbesondere in kosmetischen Formulierungen.

Somit sind Emulgatoren enthaltend erfindungsgemäße Polyglycerinester oder Polyglycerinester erhältlich bzw. erhalten nach dem erfindungsgemäßen Verfahren Gegenstand der vorliegenden Erfindung. Als Emulgator ist im Rahmen dieser Erfindung ein Emulgator zu verstehen, der mindestens aus einem erfindungsgemäßen Polyglycerinester oder Polyglycerinester erhältlich bzw. erhalten nach dem erfindungsgemäßen Verfahren und gegebenenfalls mindestens einem Co-Emulgator besteht, wobei die Anwesenheit eines Co-Emulgators bevorzugt ist.

Als Co-Emulgator können vorteilhaft folgende Co-Emulgatoren eingesetzt werden: Polyglyceryl-3 Dicitrate/Stearate, Polyglyceryl-3 Methylglucose Distearate, Polyglyceryl-10 Stearate, Polyglyceryl-6 Distearate, Methylglucose Sesquistearate, Sodium Stearoyl Glutamate, Sodium Cetearyl Sulfate, Potassium Cetyl Phosphate, Glyceryl Stearate Citrate, Cetearyl Glucoside, Glyceryl Stearate, Glyceryl Stearate SE, Cetearyl Alcohol und Stearic Acid.

Ebenso eignen sich erfindungsgemäße Polyglycerinester und Polyglycerinester erhältlich bzw. erhalten nach dem erfindungsgemäßen Verfahren hervorragend zur Verwendung zur Herstellung von kosmetischen oder pharmazeutischen Formulierungen, insbesondere zur Herstellung kosmetischer Cremes und Lotionen. Unter Cremes und Lotionen wird in diesem Zusammenhang kosmetische O/W Emulsionen mit streichfähig-pastöser bzw. fließfähiger Konsistenz verstanden. Allgemein können die erfindungsgemäßen Polyglycerinester beispielsweise in Pflegecremes und -lotionen für Gesicht, Körper und Hände, in Sonnenschutzemulsionen, in Seren, in BB Cremes, in Deodorants, in Schminken, in Aersolen, Roll-ons, Pumpsprays, Stiften z.B. im AP/Deo-Bereich, in Babypflegeprodukten, in Intimpflege-, Fußpflege-, Haarpflege-, Nagelpflege, Zahnpflege- oder Mundpflegeprodukten sowie in dermatologischen Salben eingesetzt werden.

Somit sind ebenfalls kosmetischen oder pharmazeutischen Formulierungen, insbesondere O/W Formulierungen, enthaltend erfindungsgemäße Polyglycerinester oder Polyglycerinester erhältlich bzw. erhalten nach dem erfindungsgemäßen Verfahren Gegenstand der vorliegenden Erfindung. Erfindungsgemäß bevorzugte Formulierungen stellen Sonnenschutzpräparate, elektrolythaltige Emulsionen, Formulierungen mit kosmetischen Wirkstoffen und O/W Make-Up Formulierungen dar.

Erfindungsgemäß bevorzugte Formulierungen, enthalten den erfindungsgemäßen Polyglycerinester oder Polyglycerinester erhältlich bzw. erhalten nach dem erfindungsgemäßen Verfahren in Mengen von 0.01 bis 10 Gew.%, bevorzugt 0.05 bis 8 Gew.% und besonders bevorzugt 0.1 bis 5 Gew.% bezogen auf die Gesamtformulierung.

Insbesondere kosmetische oder pharmazeutische Formulierungen sind bevorzugt, die im Wesentlichen frei von alkoxylierten Verbindungen sind. Unter dem Begriff "im Wesentlichen frei von alkoxylierten Verbindungen" im Zusammenhang mit der vorliegenden Erfindung ist zu verstehen, dass die Formulierungen keine nennenswerten Mengen an alkoxylierten Verbindungen aufweisen, die eine oberflächenaktive Wirkung ausüben. Insbesondere ist hierunter zu verstehen, dass diese Verbindungen in Mengen von kleiner 1 Gew.-%, bevorzugt von kleiner 0,1 Gew.-%, besonders bevorzugt von kleiner 0,01 Gew.-% bezogen auf die Gesamtformulierung, insbesondere keine nachweisbaren Mengen, enthalten sind.

Die erfindungsgemäßen Formulierungen können z.B. mindestens eine zusätzliche Komponente enthalten, ausgewählt aus der Gruppe der

Emollients,
Co-Emulgatoren,
Verdicker/Viskositätsregler/Stabilisatoren,
Antioxidantien,
Hydrotrope (oder Polyole),
Fest- und Füllstoffe,
Perlglanzadditive,

Deodorant- und Antitranspirantwirkstoffe,
Insektrepellentien,
Selbstbräuner,
Konservierungsstoffe,
Konditioniermittel,
Parfüme,
Farbstoffe,
Pigmente
kosmetische Wirkstoffe,
Pflegeadditive,
Überfettungsmittel,
Lösungsmittel,
UV-Filter,
Elektrolyte,
Multifunktionelle Additive,
feuchtigkeitsspendende Stoffe.

Substanzen, die als beispielhafte Vertreter der einzelnen Gruppen eingesetzt werden können, sind dem Fachmann bekannt und können beispielsweise der deutschen Anmeldung DE 102008001788.4 entnommen werden. Diese Patentanmeldung wird hiermit als Referenz eingeführt und gilt somit als Teil der Offenbarung.
Bezüglich weiterer fakultativer Komponenten sowie deren eingesetzten Mengen dieser Komponenten wird ausdrücklich auf die dem Fachmann bekannten einschlägigen Handbücher, z. B. K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung.
Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.
Da die erfindungsgemäßen Polyglycerinester Pigmente oder Festkörper überaus stabil in Emulsionspräparaten halten können, sind Fest- und Füllstoffe, insbesondere Partikel und Additive, die zur Erzielung eines spezifischen Hautgefühls eingesetzt werden, wie z.B. Siliconelastomere, PMMA-Partikel, PE-Partikel, PS-Partikel, Nylon-Partikel, Bornitrid, Stärke, Cellulose, Mica und Talkum, eine bevorzugte zusätzliche Komponente.
Erfindungsgemäße Emulgatoren sind besonders gut geeignet zur Stabilisierung niedrigviskoser Formulierungen (Lotionen) mit leichtem Hautgefühl. Besonders stark ist der Vorteil bei gleichzeitigem Vorhandensein von zusätzlichen emulsionsbelastenden Formulierungsbestandteilen.
Ganz besonders vorteilhaft geeignet ist die vorliegende Erfindung zur Stabilisierung insbesondere niedrigviskoser Sonnenschutzsysteme bei Anwesenheit von UV-Filtern.
Daher enthält eine bevorzugte kosmetische oder pharmazeutische Formulierung als zusätzliche Komponente eine Substanz ausgewählt aus der Gruppe der Substanzen der UV-Lichtschutzfilter. Diese können anorganische und organische, wasser- und öllösliche UV-Filter darstellen, wobei die wasserlöslichen besonders bevorzugt enthalten sind.
Als UV-Filter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:

3-Benzylidencampher und dessen Derivate, z.B. 3-(4-Methylbenzyliden)campher, 4-Aminobenzoesäurederivate, wie z.B. 4-(Dimethylamino)benzoesäure-2-ethylhexylester,4-(Dimethylamino)benzoesäure-2-ethylhexylester und 4-(Dimethylamino)benzoesäureamylester
Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene),
Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon,
Ester der Benzalmalonsäure, wie z.B. 4-Methoxybenzalmalonsäuredi-2-ethylhexylester,
Triazinderivate, wie z.B. 2,4,6-Trianilino-(p-carbo-2'-ethyl-1'-hexyloxy)-1,3,5-triazin und Octyltriazon,
Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage:

2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenon, wie z.B. 2-Hydroxy-4-methoxybenzophenon-5-sulfonsäure und ihre Salze, Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Besonders bevorzugte wasserlösliche UV-Filter sind beispielsweise Produkte wie Eusolex® 232 (Phenylbenzimidazole Sulfonic Acid) (Merck KGaA) oder Mexoryl® SX (Terephtalylidene Dicamphor Sulfonic Acid) (Chimex SA).

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1,1,3,3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen.

Ebenso ganz besonders vorteilhaft geeignet ist die vorliegende Erfindung zur Stabilisierung Insektrepellentien enthaltender Formulierungen, insbesondere in Verbindung mit UV-Lichtschutzfiltern.

Daher enthält eine bevorzugte kosmetische oder pharmazeutische Formulierung als zusätzliche Komponente eine Substanz ausgewählt aus der Gruppe der Substanzen der Insektrepellentien, insbesondere bevorzugt als zusätzlich mindestens eine weitere zusätzliche Komponente eine Substanz ausgewählt aus der Gruppe der Substanzen der UV-Lichtschutzfilter.

Insektrepellentien in Zusammenhang mit der vorliegenden Erfindung sind insbesondere ausgewählt aus der Gruppe bestehend aus N,N-Diethyl-m-toluamid (DEET), Ethylbutylacetylaminopropionat (z.B. IR3535 von Merck Co.), Hydroxyethylisobutylpiperidincarboxylat (1-Piperidincarboxylsäure, z.B. Bayer KBR3023), Citronella-Öl, Sojabohnenöl, Zitronengrasöl, Geraniumöl, Dimethylphtalat (DMP), Geraniolöl und p-Methan-3,8-diol oder Mischungen davon, wobei N,N-Diethyl-m-toluamid, Ethylbutylacetylaminopropionat, Hydroxyethylisobutylpiperidincarboxylat und Citronella-Öl besonders bevorzugt sind.

Die Verwendung des Emulgators in Formulierungen mit Elektrolyten oder ionischen Verbindungen ist ebenfalls ein vorteilhaftes Anwendungsgebiet. Sogenannte multifunktionelle Additive, welche unter anderem feuchtigkeitsspendende, konservierende, viskositätsregulierende, emulgierende, stabilisierende Wirkung haben können, sind mit dem erfindungsgemäßen Emulgator ebenfalls sehr gut in O/W Emulsionen, besonders in Lotionen, zu formulieren, weshalb Vertreter dieser Stoffgruppe ebenfalls eine bevorzugte zusätzliche Komponente darstellen.

Da die erfindungsgemäßen Polyglycerinester in der Lage sind, Emulsionen auch ohne die Verwendung von polyacrylatbasierten Verdickern zu stabilisieren, sind für sie PEG- und oder silikon- und oder polyacrylat- und oder paraben-freie Formulierungen auf Basis natürlicher Inhaltsstoffe ein besonders bevorzugtes Anwendungsgebiet.

Aufgrund seiner feuchtigkeitsspendenden Eigenschaften, seines leichten Hautgefühls und der guten Stabilisierung kosmetischer Wirkstoffe eignen sich erfindungsgemäße Polyglycerinesters beispielsweise bevorzugt für die Verwendung in kosmetischen Emulsionen zur Steigerung der Hautfeuchtigkeit, Verbesserung der Hautstruktur, Verringerung altersbedingter Hautveränderungen, Nivellierungs des Hauttaints, Förderung des Hautstoffwechsels oder Neutralisierung schädlicher Stoffwechselbestandteile in der Haut. Daher sind feuchtigkeitsspende Stoffe und Stoffe mit biologischer Wirksamkeit, wie Glycerin, Harnstoff, Peptide, bevorzugte zusätzliche Komponenten in bevorzugten erfindungsgemäßen Formulierungen.

Aufgrund der Toleranz der erfindungsgemäßen Emulgatoren gegenüber verschiedenartigen Konservierungsmitteln sind neben parabenhaltigen Konservierungsmitteln auch parabenfreie Konservierungsmittel wie Phenoxyethanol, Ethanol oder Methylisothiazolinon gut einsetzbar. Zur Herstellung von Naturkosmetik sind als Konservierungsmittel vor allem natürliche Säuren wie Benzoesäure, Caprylsäure, Caprinsäure, Anissäure, Sorbitansäure, Lävulinsäure, Sorbinsäure und Benzoesäure oder Stoffe wie Caprylyl Glycol bevorzugte Konservierungsmittel, die als zusätzliches Formulierungsbestandteil eingesetzt werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Abbildungen sind Bestandteil der Beispiele:

Abbildung 1: GPC-Chromatogramm der Polyglycerinfraktion eines erfindungsgemäßen Polyglycerinesters mit besonderer Oligomerenverteilung des Polyglycerins nach alkalischer Hydrolyse und Abtrennung der Fettsäuren gemäß Beispiel 1.
Abbildung 2: GPC-Chromatogramm der Polyglycerinfraktion eines nicht erfindungsgemäßen Polyglycerinesters mit normaler Oligomerenverteilung nach alkalischer Hydrolyse und Abtrennung der Fettsäuren gemäß Beispiel 5.

Beispiele:

*Beispiel 1:*

Ein Gemisch aus Glycerin (2102 g, 22,8 Mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (360,0 g, 0,45 Mol) und Glycerin (47,82 g, 0,52 Mol) mit einer Hydroxylzahl von 1000 mg KOH/g sowie einem Gemisch aus Stearinsäure und Palmitinsäure im Verhältnis 1:1 (334,19 g, 1,24 Mol), Behensäure (28,0 g, 0,08 Mol) und $Na_2CO_3$ (3,75 g, 0,035 Mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war.

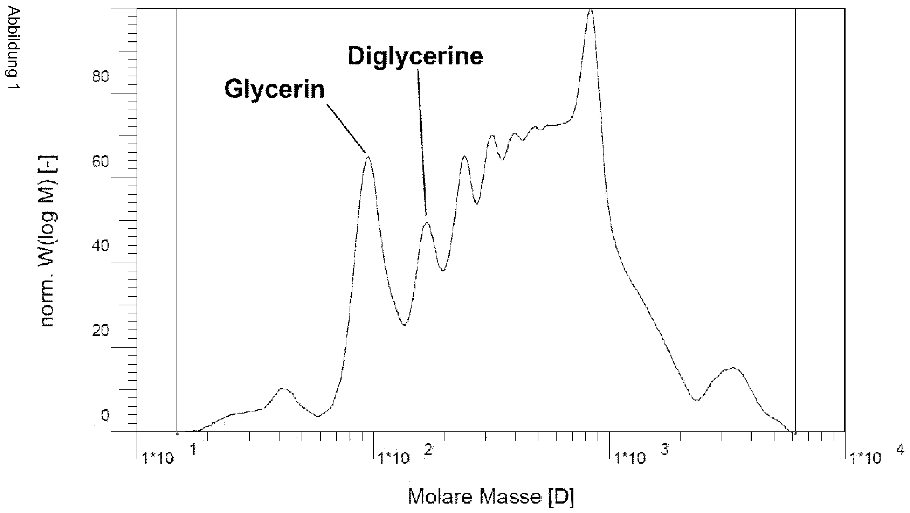

Ein GPC-Chromatogramm des so erhaltenen Polyglycerinesters nach dessen alkalischer Spaltung und Abtrennung der Fettsäuren ist in Abbildung 1 dargestellt.

*Beispiel 2:*

Ein Gemisch aus Glycerin (2102 g, 22,8 Mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (310,5 g, 0,38 Mol) und Glycerin (41,25 g, 0,45 Mol) mit einer Hydroxylzahl von 1000 mg KOH/g sowie Behensäure (481,9 g, 1,40 Mol) und $Na_2CO_3$ (4,17 g, 0,039 Mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1,0 erreicht war.

*Beispiel 3:*

Ein Gemisch aus Glycerin (2102 g, 22,8 Mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.4900 (typischerweise nach 20-21 h bei 240 °C) erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 880 mg KOH/g aufwies.

Ein Gemisch aus dem so erhaltenen Polyglycerin (337,8 g, 0,42 Mol) und Glycerin (44,87 g, 0,49 Mol) mit einer Hydroxylzahl von 1000 mg KOH/g sowie einem Gemisch aus Stearinsäure und Palmitinsäure im Verhältnis 1:1 (334,19 g, 1,24 Mol) und $Na_2CO_3$ (3,58 g, 0,034 Mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1,0 erreicht war.

*Beispiel 4:*

Ein Gemisch eines Polyglycerinesters erhalten wie beschrieben in Beispiel 2 (5,9 g) und eines Polyglycerinesters erhalten wie beschrieben in Beispiel 3 (89,9 g) wurde auf 75 °C erhitzt und für 1 h bei dieser Temperatur gerührt.

*Beispiel 5 (nicht erfindungsgemäß):*

Ein Gemisch aus Glycerin (2102 g, 22,8 Mol) und 45%iger wässriger Kalilauge (24,2 g) wurde bei 400 mbar innerhalb von 1 Stunde auf 240 °C erhitzt und das entstehende Wasser kontinuierlich abdestilliert. Sobald das Reaktionsgemisch einen Brechungsindex von ≥1.485 erreicht hatte, wurde der Druck langsam auf 50 mbar gesenkt und weiter Wasser sowie überschüssiges Glycerin bei 240 °C abdestilliert, bis das verbleibende Gemisch eine Hydroxylzahl von 1000 mg KOH/g und ein Massenverhältnis von Glycerin zu Diglycerin von 0,3 aufwies.

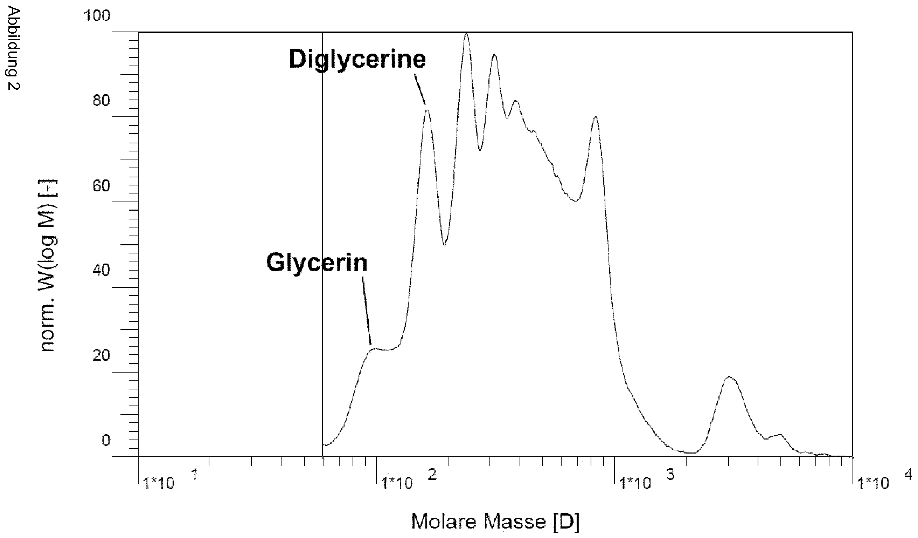

Ein Gemisch aus dem so erhaltenen Polyglycerin (407,82 g, 1,00 Mol) und einem Gemisch aus Stearinsäure und Palmitinsäure im Verhältnis 1:1 (334,19 g, 1,24 Mol), Behensäure (28,0 g, 0,08 Mol) und $Na_2CO_3$ (3,75 g, 0,035 Mol) wurde innerhalb von 3 h unter Stickstoffeinleitung bis auf 240 °C erhitzt und das Gemisch anschließend so lange bei dieser Temperatur gerührt und das entstehende Wasser kontinuierlich entfernt, bis eine Säurezahl von ≤1.0 erreicht war. Ein GPC-Chromatogramm des so erhaltenen Polyglycerinesters nach dessen alkalischer Spaltung und Abtrennung der Fettsäuren ist in Abbildung 2 dargestellt

*Beispiel 6: Einfluss der besonderen Oligomerenverteilung des Polyglycerins Differenzierung der Performance gegen den Stand der Technik*

Alle Konzentrationen in den Anwendungsbeispielen sind in Gewichtsprozent angegeben. Zur Herstellung der Emulsionen wurden dem Fachmann bekannte übliche Homogenisierverfahren eingesetzt. Die Herstellung der Emulsionen erfolgte daher typischerweise so, dass Öl- und Wasserphase auf 70 - 75°C erwärmt wurden. Anschließend wurde entweder die Ölphase in die Wassereingerührt oder Öl- und Wasserphase ohne Rühren zusammengegeben. Anschließend wurde mit einem geeigneten Homogenisator (z.B. Ultraturrax) für ca. 1-2 Minuten homogenisiert. Stabilisierende Polymere (z.B. Carbomere) werden bevorzugt als Öldispersion bei Temperaturen von 50 - 60°C in die Emulsion eingerührt. Anschließend wird kurz homogenisiert. Zugabe weiterer Inhaltsstoffe (z.B. Konservierungsmittel, Wirkstoffe) erfolgte bevorzugt bei 40°C. Falls die Rezepturen mit organischen Säuren konserviert wurden, wurde der pH-Wert der Emulsionen auf ca. 5 eingestellt.

Diese Versuche sollen zeigen, dass die erfindungsgemäßen Polyglycerinester Vorteile in Bezug auf Emulsionsstabilität aufweisen. Als Repräsentant für auf dem Stand der Technik beruhenden polglycerinbasierten O/W Emulgatoren wurde dabei der nicht erfindungsgemäße Emulgator aus Beispiel 5 gewählt. Zur Überprüfung der Lagerstabilität der Emulsionen wurden diese drei Monate bei Raumtemperatur, 40°C und 45°C gelagert. Zur Überprüfung der Kältestabilität wurde außerdem ein Monat lang bei -5°C gelagert und drei Gefrier-Tau-Zyklen von 25°C/-15°C/25°C durchgeführt. Deutlich Veränderungen im Erscheinungsbild oder der Konsistenz sowie insbesondere Öl- oder Wasserabscheidungen wurden als Kriterien für Instabilität gewichtet.

Vergleich der erfindungsgemäßen Emulgatoren 1 und 4 gegen den nicht erfindungsgemäßen Emulgator 5 in verschiedenen Rezepturen: 1 - Lotion mit natürlichem Konservierungsmittel, 2 - Lotion mit Elektrolytzusatz, 3 - Lotion mit Zusatz von wasserlöslichem UV-Filter.

| Rezeptur | 1-1 | 1-2 | V1 |
|---|---|---|---|
| Emulgator 1 | 3,0% | | |
| Emulgator 4 | | 3,0% | |
| Emulgator 5 (nicht erfind.) | | | 3,0% |
| Glyceryl Stearate | 0,4% | 0,4% | 0,4% |
| Stearyl Alcohol | 0,4% | 0,4% | 0,4% |
| C12-15 Alkyl Benzoate | 3,7% | 3,7% | 3,7% |
| Caprylic/Capric Triglyceride | 3,7% | 3,7% | 3,7% |
| Isopropyl Palmitate | 3,7% | 3,7% | 3,7% |
| Glycerin | 5,0% | 5,0% | 5,0% |
| Demineralized Water | ad 100% | ad 100% | ad 100% |
| Xanthan Gum [1)] | 0,5% | 0,5% | 0,5% |
| Sodium Hydroxide (10% aq., pH-Wert-Einstellung auf 5,0) | q.s. | q.s. | q.s. |
| Benzyl Alcohol, Benzoic Acid, Sorbic Acid [2)] | 1,0% | 1,0% | 1,0% |
| | | | |
| Konsistenz nach Herstellung | flüssig-pastös | flüssig-pastös | flüssig-pastös |
| Stabilität | Stabil | Stabil | Wasserseparation nach 1 Monat 45°C |

(fortgesetzt)

| Rezeptur | 2-1 | 2-2 | V2 |
|---|---|---|---|
| Emulgator 1 | 3,0% | | |
| Emulgator 4 | | 3,0% | |
| Emulgator 5 (nicht erfind.) | | | 3,0% |
| Glyceryl Stearate | 0,4% | 0,4% | 0,4% |
| Stearyl Alcohol | 0,4% | 0,4% | 0,4% |
| C12-15 Alkyl Benzoate | 5,4% | 5,4% | 5,4% |
| Caprylic/Capric Triglyceride | 5,4% | 5,4% | 5,4% |
| Isopropyl Palmitate | 5,4% | 5,4% | 5,4% |
| Glycerin | 5,0% | 5,0% | 5,0% |
| Sodium Chloride | 1,0% | 1,0% | 1,0% |
| Demineralized Water | ad 100% | ad 100% | ad 100% |
| Xanthan Gum [1] | 0,5% | 0,5% | 0,5% |
| Methylisothiazolinone, Methylparaben, Ethylparaben; Dipropylene Glycol [3] | 0,8 | 0,8 | 0,8 |
| | | | |
| Konsistenz nach Herstellung | flüssig-pastös | flüssig-pastös | flüssig-pastös |
| Stabilität | Stabil | Stabil | Wasserseparation nach 1 Monat 45°C |
| **Rezeptur** | **3-1** | **3-2** | **V3** |
| Emulgator 1 | 4,0% | | |
| Emulgator 4 | | 4,0% | |
| Emulgator 5 (nicht erfind.) | | | 4,0% |
| Glyceryl Stearate | 0,5% | 0,5% | 0,5% |
| Stearyl Alcohol | 0,5% | 0,5% | 0,5% |
| C12-15 Alkyl Benzoate | 5,0% | 5,0% | 5,0% |
| Caprylic/Capric Triglyceride | 5,0% | 5,0% | 5,0% |
| Isopropyl Palmitate | 5,0% | 5,0% | 5,0% |
| Glycerin | 5,0% | 5,0% | 5,0% |
| Demineralized Water | ad 100% | ad 100% | ad 100% |
| Xanthan Gum [1] | 0,3% | 0,3% | 0,3% |
| UV-Filterlösung [4] | 20,0% | 20,0% | 20,0% |
| Methylisothiazolinone, Methylparaben, Ethylparaben; Dipropylene Glycol [3] | 0,8% | 0,8% | 0,8% |
| | | | |
| Konsistenz nach Herstellung | dünnflüssig bis mittelviskos | dünnflüssig bis mittelviskos | dünnflüssig |

(fortgesetzt)

| Rezeptur | 3-1 | 3-2 | V3 |
|---|---|---|---|
| Stabilität | Stabil | Stabil | Wasserseparation nach 1 Monat 45°C |

<table>
<tr><td>

[1] Keltrol CG-SFT (CP Kelco)

[2] Optiphen BSB-N / Rokonsal BSB-N (Ashland Specialty Ingredients)

[3] Microcare® MEM (Thor)

[4] 10% Phenylbenzimidazole Sulfonic Acid, 1,4% Natriumhydroxid, ad 100% Wasser

</td></tr>
</table>

Während die Rezepturen mit den erfindungsgemäßen Emulgatoren 1 und 4 jeweils zu einer lagerstabilen Formulierung führt, zeigen die Cremes mit dem nicht erfindungsgemäßen Vergleichsemulgator aus Beispiel 5 deutliche Schwächen in der Lagerstabilität.

*Vergleich erfindungsgemäßer Emulgatoren gegen nicht erfindungsgemäße Emulgatoren in einer Sonnenschutzlotion in Kombination mit Insektenschutzmittel.*

Die nicht erfindungsgemäßen Emulgatoren sind typische Vertreter, welche in marktüblichen Produkten dieses Anwendungstyps eingesetzt werden. Die jeweiligen Emulgatoren wurden in den Formulierungen mit marktüblichen Einsatzkonzentrationen verwendet, wobei die Viskosität der Emulsionen durch Variation der Konsistenzgeber einheitlich auf die Viskosität einer dünnflüssigen Lotion eingestellt wurde.

| Rezeptur | 4-1 usw. | Z-2 | Z-V1 | Z-V2 |
|---|---|---|---|---|
| Emulgator 1 | 3.0 | - | - | - |
| Emulgator 4 | - | 3.0 | - | - |
| Polyglyceryl-3 Methylglucose Distearate [a] | - | - | 3.0 | - |
| Polyglyceryl-3 Dicitrate/ Stearate [b] | - | - | - | 3.0 |
| Glyceryl Stearate | 0.5 | 0.5 | - | - |
| Stearyl Alcohol | 0.5 | 0.5 | - | - |
| Phenoxyethyl Caprylate [c] | 2.3 | 2.3 | 3.3 | 3.3 |
| Butyl Methoxydibenzoylmethane | 5.0 | 5.0 | 5.0 | 5.0 |
| Diethylhexyl Butamido Triazone | 0.7 | 0.7 | 0.7 | 0.7 |
| Ethylhexyl Salicylate | 0.5 | 0.5 | 0.5 | 0,5 |
| Octocrylene | 6.0 | 6.0 | 6.0 | 6.0 |
| Bis-Ethylhexyloxyphenol Methoxyphenyl Triazine [d] | 1.5 | 1.5 | 1.5 | 1.5 |
| Ethyl Butylacetylaminopropionate [e] | 4.0 | 4.0 | 4.0 | 4.0 |
| Tocopheryl Acetate | 0.5 | 0.5 | 0.5 | 0.5 |
| Nylon 10/10 [f] | 0.5 | 0.5 | 0.5 | 0.5 |
| Wasser | Ad 100 | Ad 100 | Ad 100 | Ad 100 |
| Glycerin | 2.0 | 2.0 | 2.0 | 2.0 |
| Hydroxyethylcellulose [g] | 0.5 | 0.5 | 0.5 | 0.5 |

(fortgesetzt)

| Rezeptur | 4-1 usw. | Z-2 | Z-V1 | Z-V2 |
|---|---|---|---|---|
| Methylene Bis-Benzotriazolyl Tetramethylbutylphenol (50% aq.) [h] | 11.0 | 11.0 | 11.0 | 11.0 |
| Dipropylene Glycol; Methylparaben; Ethylparaben; Aqua; Methylisothiazolinone [3] | 0.8 | 0.8 | 0.8 | 0.8 |
| Konsistenz nach Herstellung | Niedrigviskose Lotion | Niedrigviskose Lotion | Niedrigviskose Lotion | Niedrigviskose Lotion |
| Stabilität | Stabil | Stabil | Ölseparation nach 2 Wochen Lagerung bei 45°C | Ölseparation nach 2 Wochen Lagerung bei 45°C |

a) entspricht [27]
b) entspricht [21]
c) TEGOSOFT XC (Evonik Industries AG) entspricht [9]
d) (Tinosorb S, BASF SE) entspricht [8]
e) (Insektenschutzmittel IR 3535, Merck)
f) (TEGOLON ECO 10-10, Evonik Industries AG) entspricht [33]
g) (Natrosol HHR 250, Ashland Specialty Chemicals)
h) (Tinosorb M, BASF SE) entspricht [39]

| Rezeptur | Z-V3 | Z-V4 | Z-V5 | Z-V6 |
|---|---|---|---|---|
| Glyceryl Stearate Citrate [I] | 2.0 | - | - | - |
| Polyglyceryl-10 Stearate [J] | - | 3.0 | - | - |
| Polyglyceryl-6 Distearate [K] | - | - | 3.0 | - |
| Cetearyl Glucoside [l] | - | - | - | 1.5 |
| Glyceryl Stearate | 1.0 | 1.5 | 0.5 | 0.8 |
| Stearyl Alcohol | 1.0 | 1.3 | 0.5 | 1.0 |
| Phenoxyethyl Caprylate [c] | 2.3 | 0.5 | 2.3 | 3.0 |
| Butyl Methoxydibenzoylmethane | 5.0 | 5.0 | 5.0 | 5.0 |
| Diethylhexyl Butamido Triazone | 0.7 | 0.7 | 0.7 | 0.7 |
| Ethylhexyl Salicylate | 0.5 | 0,5 | 0.5 | 0.5 |
| Octocrylene | 6.0 | 6.0 | 6.0 | 6.0 |
| Bis-Ethyl hexyloxyphenol Methoxyphenyl Triazine [d] | 1.5 | 1,5 | 1.5 | 1.5 |
| Ethyl Butylacetylaminopropionate [e] | 4.0 | 4.0 | 4.0 | 4.0 |
| Tocopheryl Acetate | 0.5 | 0.5 | 0.5 | 0.5 |
| Nylon 10/10 [f] | 0.5 | 0.5 | 0.5 | 0.5 |
| Wasser | Ad 100 | Ad 100 | Ad 100 | Ad 100 |

(fortgesetzt)

| Rezeptur | Z-V3 | Z-V4 | Z-V5 | Z-V6 |
|---|---|---|---|---|
| Glycerin | 2.0 | 2.0 | 2.0 | 2.0 |
| Hydroxyethylcellulose [g] | 0.5 | 0,5 | 0.5 | 0.5 |
| Methylene Bis-Benzotriazolyl Tetramethylbutylphenol (50% aq.) [h] | 11.0 | 11.0 | 11.0 | 11.0 |
| Dipropylene Glycol; Methylparaben; Ethylparaben; Aqua; Methylisothiazolinone [3] | 0.8 | 0.8 | 0.8 | 0.8 |
| Konsistenz nach Herstellung | Niedrigviskose Lotion | Niedrigviskose Lotion | Niedrigviskose Lotion | Niedrigviskose Lotion |
| Stabilität | Ölseparation nach 2 Wochen Lagerung bei 45°C | Ölseparation nach 2 Wochen Lagerung bei 45°C; Viskositätsanstieg (pastöse Konsistenz nach Monat Lagerung bei RT) | Ölseparation nach 2 Tagen Lagerung bei 45°C | Ölseparation nach 2 Wochen Lagerung bei 45°C |
| [i] entspricht [7] | | | | |
| [j] Polyaldo 10-1-S KFG (Lonza) | | | | |
| [k] Plurol Stearique WL 1009 (Gattefosse sas) | | | | |
| [l] entspricht [5] | | | | |

| Rezeptur | Z-V7 | Z-V8 | Z-V9 |
|---|---|---|---|
| Sodium Stearoyl Glutamate [m] | 1.5 | - | - |
| Sodium Cetearyl Sulfate [n] | - | 1.0 | - |
| Potassium Cetyl Phosphate [o] | - | - | 1.5 |
| Glyceryl Stearate | 1.0 | 0.4 | 0.6 |
| Stearyl Alcohol | 1.2 | 0.4 | 0.6 |
| Phenoxyethyl Caprylate [c] | 2.6 | 4.5 | 3.6 |
| Butyl Methoxydibenzoylmethane | 5.0 | 5.0 | 5.0 |
| Diethylhexyl Butamido Triazone | 0.7 | 0.7 | 0.7 |
| Ethylhexyl Salicylate | 0,5 | 0.5 | 0,5 |
| Octocrylene | 6.0 | 6.0 | 6.0 |
| Bis-Ethylhexyloxyphenol Methoxyphenyl Triazine [d] | 1.5 | 1.5 | 1,5 |
| Ethyl Butylacetylaminopropionate [e] | 4.0 | 4.0 | 4.0 |
| Tocopheryl Acetate | 0.5 | 0.5 | 0.5 |
| Nylon 10/10 [f] | 0.5 | 0.5 | 0.5 |

(fortgesetzt)

| Rezeptur | Z-V7 | Z-V8 | Z-V9 |
|---|---|---|---|
| Wasser | Ad 100 | Ad 100 | Ad 100 |
| Glycerin | 2.0 | 2.0 | 2.0 |
| Hydroxyethylcellulose [9] | 0.5 | 0.5 | 0,5 |
| Methylene Bis-Benzotriazolyl Tetramethylbutylphenol (50% aq.) [h] | 11.0 | 11.0 | 11.0 |
| Dipropylene Glycol; Methylparaben; Ethylparaben; Aqua; Methylisothiazolinone [3] | 0.8 | 0.8 | 0.8 |
| Konsistenz nach Herstellung | Niedrigviskose Lotion | Niedrigviskose Lotion | Niedrigviskose Lotion |
| Stabilität | Ölseparation nach 2 Wochen Lagerung bei 45°C | Ölseparation nach 2 Wochen Lagerung bei 45°C bzw. 40°C. Phasentrennung nach 1 Monat Lagerung bei -5°C bzw. nach 3 Zyklen -15°C/RT | Ölseparation nach 2 Wochen Lagerung bei 45°C bzw. 40°C Ölseparation nach 1 Monat Lagerung bei - 5°C |
| [m] entspricht [12] | | | |
| [n] entspricht [16] | | | |
| [o] Amphisol K (DSM Nutritional Products Inc.) | | | |

Während die Rezepturen Z-1 und Z-2 mit den erfindungsgemäßen Emulgatoren 1 und 4 jeweils zu einer lagerstabilen Formulierung führt, zeigen die Lotionen mit den nicht erfindungsgemäßen Vergleichsemulgatoren (Z-V1 bis Z-V9) eindeutige Schwächen in der Lagerstabilität.

*Formulierungsbeispiele*

Diese Beispiele sollen zeigen, dass die erfindungsgemäßen Polyglycerinester in einer Vielzahl kosmetischer Formulierungen eingesetzt werden können.
Es ist darüber hinaus mit Hilfe der erfindungsgemäßen Polyglycerinester möglich, Pigmente oder Festkörper stabil in Emulsionspräparate einzuarbeiten.
Weiterhin zeigen die Beispiele die gute Kompatibilität mit typischen Coemulgatoren, Ölen, Verdickern und Stabilisatoren sowie die gute Verträglichkeit mit emulsionsbelastenden Inhaltsstoffen wie UV-Filtern, antimikrobiellen Wirkstoffen oder kosmetischen Wirkstoffen.

| Rezeptur | 4-1 | 4-2 | 4-3 |
|---|---|---|---|
| Emulgator 1 | 3,0% | | 2,5% |
| Emulgator 4 | | 3,0% | |
| Cetearyl Glucoside [5] | | | 0,5% |
| Glyceryl Stearate | 0,2% | 0,2% | 0,2% |
| Stearyl Alcohol | 0,2% | 0,2% | 0,2% |
| Prunus Amygdalus Dulcis (Sweet Almond) Oil | 10,0% | 10,0% | 10,0% |
| Isoamyl Cocoate [6] | 6,6% | 6,6% | 6,6% |
| Glycerin | 4,0% | 4,0% | 4,0% |
| Demineralized Water | ad 100% | ad 100% | ad 100% |
| Xanthan Gum [1] | 0,5% | 0,5% | 0,5% |

(fortgesetzt)

| Rezeptur | 4-1 | 4-2 | 4-3 |
|---|---|---|---|
| Sodium Hydroxide (10% aq.)(pH-Wert-Einstellung auf 5,0) | q.s. | q.s. | q.s. |
| Benzyl Alcohol, Benzoic Acid, Sorbic Acid [2] | 1,0% | 1,0% | 1,0% |
| [5] TEGO Care CG 90 (Evonik Industries AG)<br>[6] TEGOSOFT AC (Evonik Industries AG) | | | |

Sonnenschutzspray

| Rezeptur | 5-1 | 5-2 | 5-3 |
|---|---|---|---|
| Emulgator 1 | 3,0% | | |
| Emulgator 4 | | 3,0% | 2,5% |
| Glyceryl Stearate Citrate [7] | | | 0,5% |
| Glyceryl Stearate | 0,5% | 0,5% | 0,5% |
| Stearyl Alcohol | 0,5% | 0,5% | 0,5% |
| Bis-Ethylhexyloxyphenol Methoxyphenyl Triazine [8] | 3,0% | 3,0% | 3,0% |
| Butyl Methoxydibenzoylmethane | 2,0% | 2,0% | 2,0% |
| Ethylhexyl Methoxycinnamate | 2,0% | 2,0% | 2,0% |
| Ethylhexylsalicylat | 4,0% | 4,0% | 4,0% |
| Octocrylene | 4,0% | 4,0% | 4,0% |
| Isopropyl Palmitate | 2,0% | 2,0% | 2,0% |
| Phenoxyethyl Caprylate [9] | 3,2% | 3,2% | 3,2% |
| Glycerin | 3,0% | 3,0% | 3,0% |
| Demineralized Water | ad 100% | ad 100% | ad 100% |
| Carbomersuspension [10] | 1,0% | 1,0% | 1,0% |
| Tromethamin (30%ig) | 0,9% | 0,9% | 0,9% |
| UV-Filterlösung [11] | 10,0% | 10,0% | 10,0% |
| Methylisothiazolinone, Methylparaben, Ethylparaben; Dipropylene Glycol [4][3] | 0,8 | 0,8 | 0,8 |
| [7] AXOL C 62 (Evonik Industries AG)<br>[8] Tinosorb S (BASF SE)<br>[9] TEGOSOFT XC (Evonik Industries AG)<br>[10] Acrylates/C10-30 Alkyl Acrylate Crosspolymer (TEGO Carbomer 341ER) 20%ig in Phenoxyethyl Caprylate<br>[11] 20 % Phenylbenzimidazole Sulfonic Acid, 8,8% Tris (hydroxymethyl)-aminomethan, Demineralized Water ad 100% | | | |

AP/Deo Lotion

| Rezeptur | 6-1 | 6-2 | 6-3 |
|---|---|---|---|
| Emulgator 1 | 3,0% | | 2,5% |
| Emulgator 4 | | 3,0% | |
| Sodium Stearoyl Glutamate [12] | | | 0,5% |
| Diethylhexyl Carbonate [13] | 3,0% | 3,0% | 3,0% |
| PPG-14 Butyl Ether | 3,0% | 3,0% | 3,0% |

(fortgesetzt)

| Rezeptur | 6-1 | 6-2 | 6-3 |
|---|---|---|---|
| Polyglyceryl-3 Caprylate | 0,5% | 0,5% | 0,5% |
| Demineralized Water | ad 100% | ad 100% | ad 100% |
| Hydroxyethylcellulose [14] | 1,0% | 1,0% | 1,0% |
| Aluminum Chlorohydrate (50%ig) [15] | 15,0% | 15,0% | 15,0% |
| Methylisothiazolinone, Methylparaben, Ethylparaben; Dipropylene Glycol [3] | 0,8% | 0,8% | 0,8% |
| [12] Eumulgin SG (BASF SE)  [13] TEGOSOFT DEC (Evonik Industries AG)  [14] Natrosol 250 HHR (Ashland Specialty Chemicals)  [15] Reach 501L (Reheis) | | | |

AP/Deo Creme

| Rezeptur | 7-1 | 7-2 | 7-3 |
|---|---|---|---|
| Emulgator 1 | 3,0% | | |
| Emulgator 4 | | 3,0% | 2,5% |
| Sodium Cetearyl Sulfate [16] | | | 0,5% |
| Glyceryl Stearate | 1,0% | 1,0% | 1,0% |
| Stearyl Alcohol | 1,0% | 1,0% | 1,0% |
| PPG-15 Stearyl Ether | 5,0% | 5,0% | 5,0% |
| Isoamyl Cocoate [6] | 5,0% | 5,0% | 5,0% |
| Diethylhexyl Carbonate [13] | 5,0% | 5,0% | 5,0% |
| Persea Gratissima (Avocado) Oil | 2,0% | 2,0% | 2,0% |
| Polyglyceryl-3 Caprylate | 0,5% | 0,5% | 0,5% |
| Zinc Ricinoleate | 1,0% | 1,0% | 1,0% |
| Demineralized Water | ad 100% | ad 100% | ad 100% |
| Hydroxyethylcellulose [14] | 1,0% | 1,0% | 1,0% |
| Aluminum Chlorohydrate (50%ig) [15] | 15,0% | 15,0% | 15,0% |
| Methylisothiazolinone, Methylparaben, Ethylparaben; Dipropylene Glycol [3] | 0,8% | 0,8% | 0,8% |
| [16] Lanette E (BASF SE) | | | |

Lotionen mit kosmetischen Wirkstoffen

| Rezeptur | 8-1 | 8-2 | 8-3 | 9-1 | 9-2 | 9-3 |
|---|---|---|---|---|---|---|
| Emulgator 1 | 3,5% | | | 3,5% | | 3,0% |
| Emulgator 4 | | 3,5% | 2,5% | | 3,5% | |
| Polyglyceryl-10 Stearate | | | 0,5% | | | |
| Stearic Acid | | | | | | 0,5% |
| Glyceryl Stearate | 0,5% | 0,5% | 0,5% | 0,6% | 0,6% | 0,6% |
| Cetearyl Alcohol | 0,5% | 0,5% | 0,5% | 0,6% | 0,6% | 0,6% |
| Caprylic/Capric Triglyceride | 8,5% | 8,5% | 8,5% | 8,5% | 8,5% | 8,5% |

(fortgesetzt)

| Rezeptur | 8-1 | 8-2 | 8-3 | 9-1 | 9-2 | 9-3 |
|---|---|---|---|---|---|---|
| Ethylhexyl Palmitate [17] | 8,5% | 8,5% | 8,5% | 8,5% | 8,5% | 8,5% |
| Demineralized Water | ad 100% | ad 100% | ad 100% | ad 100% | ad 100% | ad 100% |
| Xanthan Gum [1] | 0,8% | 0,8% | 0,8% | 0,8% | 0,8% | 0,8% |
| Terminalia Arjuna Bark Extract; Pentylene Glycol (proposed) [18] | | | | 2,0% | 2,0% | 2,0% |
| Betaine; Urea; Potassium Lactate; Sodium Polyglutamate (proposed); Hydrolyzed Sclerotium Gum [19] | 5,0% | 5,0% | 5,0% | | | |
| Phenoxyethanol, Methylparaben, Ethylparaben, Propylparaben [20] | 1,0% | 1,0% | 1,0% | | | |
| Methylisothiazolinone, Methylparaben, Ethylparaben; Dipropylene Glycol [3] | | | | 0,8% | 0,8% | 0,8% |
| [17] TEGOSOFT OP (Evonik Industries AG) [18] TEGO Arjuna S (Evonik Industries AG) [19] TEGO Smooth (Evonik Industries AG) [20] Phenonip XB (Clariant International Ltd.) | | | | | | |

Lotion mit geringem Ölphasengehalt

| Rezeptur | 10-1 | 10-2 | 10-3 |
|---|---|---|---|
| Emulgator 1 | 3,0% | | 2,0% |
| Emulgator 4 | | 3,0% | |
| Polyglyceryl-3 Dicitrate/Stearate [21] | | | 1,0% |
| Cetearyl Alcohol | 0,5% | 0,5% | 0,5% |
| Caprylic/Capric Triglyceride | 6,5% | 6,5% | 6,5% |
| Demineralized Water | ad 100% | ad 100% | ad 100% |
| Xanthan Gum [1] | 0,5% | 0,5% | 0,5% |
| Methylisothiazolinone, Methylparaben, Ethylparaben; Dipropylene Glycol [3] | 0,8% | 0,8% | 0,8% |
| [21] TEGO Care PSC 3 (Evonik Industries AG) | | | |

O/W Seren 1

| Rezeptur | 11-1 | 11-2 | 11-3 | 12-1 | 12-2 |
|---|---|---|---|---|---|
| Emulgator 1 | 2,5% | | | 4,0% | |
| Emulgator 4 | | 2,5% | 2,5% | | 4,0% |
| Sodium Stearoyl Glutamate [12] | | | 1,0% | | |
| Cetearyl Glucoside [5] | | | | | |
| Polyglyceryl-3 Dicitrate/Stearate [21] | | | | | |
| Glyceryl Stearate | | | | 0,75% | 0,75% |
| Stearyl Alcohol | | | | 0,75% | 0,75% |
| Caprylic/Capric Triglyceride | 2,0% | 2,0% | 2,0% | 2,0% | 2,0% |

(fortgesetzt)

| Rezeptur | 11-1 | 11-2 | 11-3 | 12-1 | 12-2 |
|---|---|---|---|---|---|
| Oleyl Erucate [22] | 2,0% | 2,0% | 2,0% | 2,0% | 2,0% |
| Isoamyl Cocoate [6] | | | | 3,0% | 3,0% |
| Persea Gratissima (Avocado) Oil | 1,0% | 1,0% | 1,0% | 1,0% | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate [23] | 3,0% | 3,0% | 3,0% | | |
| Demineralized Water | ad 100% | ad 100% | ad 100% | ad 100% | ad 100% |
| Butylene Glycol | 5,0% | 5,0% | 5,0% | 5,0% | 5,0% |
| Tetrapeptide-21; Glycerin; Butylene Glycol; Aqua [24] | 2,0% | 2,0% | 2,0% | 2,0% | 2,0% |
| Hydrolyzed Hyaluronic Acid [25] | 0,1% | 0,1% | 0,1% | 0,1% | 0,1% |
| Xanthan Gum [1] | 0,5% | 0,5% | 0,5% | 0,5% | 0,5% |
| Sodium Hydroxide (10% aq.) | 0,2% | 0,2% | 0,2% | 0,2% | 0,2% |
| Benzyl Alcohol, Benzoic Acid, Sorbic Acid [2] | 0,8% | 0,8% | 0,8% | 0,8% | 0,8% |
| Polyglutamic acid; Sclerotium Glucan; Betaine; Urea; Potassium Lactate [26] | 3,0% | 3,0% | 3,0% | 3,0% | 3,0% |
| [22] TEGOSOFT OER (Evonik Industries AG)<br>[23] Hyacare Filler CL (Evonik Industries)<br>[24] TEGO PEP 4-17 (Evonik Industries)<br>[25] Hyacare 50 (Evonik Industries)<br>[26] TEGO Smooth Complex (Evonik Industries) | | | | | |

O/W Seren 2

| Rezeptur | 12-3 | 13-1 | 13-2 | 13-3 |
|---|---|---|---|---|
| Emulgator 1 | 3,0% | 3,0% | | 2,0% |
| Emulgator 4 | | | 3,0% | |
| Sodium Stearoyl Glutamate [12] | | | | |
| Cetearyl Glucoside [5] | 0,5% | | | |
| Polyglyceryl-3 Dicitrate/Stearate [21] | | | | 1,5% |
| Glyceryl Stearate | 0,75% | 0,5% | 0,5% | 0,5% |
| Stearyl Alcohol | 0,75% | 0,5% | 0,5% | 0,5% |
| Caprylic/Capric Triglyceride | 2,0% | 2,0% | 2,0% | 2,0% |
| Oleyl Erucate [22] | 2,0% | 2,0% | 2,0% | 2,0% |
| Isoamyl Cocoate [6] | 3,0% | | | |
| Persea Gratissima (Avocado) Oil | 1,0% | 1,0% | 1,0% | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate [23] | | 3,0% | 3,0% | 3,0% |
| Demineralized Water | ad 100% | ad 100% | ad 100% | ad 100% |

(fortgesetzt)

| Rezeptur | 12-3 | 13-1 | 13-2 | 13-3 |
|---|---|---|---|---|
| Butylene Glycol | 5,0% | 5,0% | 5,0% | 5,0% |
| Tetrapeptide-21; Glycerin; Butylene Glycol; Aqua [24] | 2,0% | 2,0% | 2,0% | 2,0% |
| Hydrolyzed Hyaluronic Acid [25] | 0,1% | 0,1% | 0,1% | 0,1% |
| Xanthan Gum [1] | 0,5% | 0,5% | 0,5% | 0,5% |
| Sodium Hydroxide (10% aq.) | 0,2% | 0,2% | 0,2% | 0,2% |
| Benzyl Alcohol, Benzoic Acid, Sorbic Acid [2] | 0,8% | 0,8% | 0,8% | 0,8% |
| Polyglutamic acid; Sclerotium Glucan; Betaine; Urea; Potassium Lactate [26] | 3,0% | | | |

O/W Blemish Balm Lotion

| Rezeptur | 14-1 | 14-2 | 14-3 |
|---|---|---|---|
| Emulgator 1 | 4,00% | | 3,00% |
| Emulgator 4 | | 4,00% | |
| Polyglyceryl-3 Methylglucose Distearate [27] | | | 1,00% |
| Glyceryl Stearate | 0,75% | 0,75% | 0,75% |
| Stearyl Alcohol | 0,75% | 0,75% | 0,75% |
| Diethylhexyl Carbonate [13] | 7,40% | 7,40% | 7,40% |
| Aqua; Ethylhexyl Stearate; Sodium Hyaluronate Crosspolymer; Polyglyceryl-4 Diisostearate/ Polyhydroxystearate/Sebacate; Sodium Isostearate [23] | 2,00% | 2,00% | 2,00% |
| Ethylhexyl Methoxycinnamate | 5,00% | 5,00% | 5,00% |
| Diethylamino Hydroxybenzoyl Hexyl Benzoate [28] | 3,00% | 3,00% | 3,00% |
| Phytosphingosine | 0,10% | 0,10% | 0,10% |
| Hydrolyzed Hyaluronic Acid [25] | 0,10% | 0,10% | 0,10% |
| Tetrapeptide-21; Glycerin; Butylene Glycol; Aqua [24] | 2,00% | 2,00% | 2,00% |
| Demineralized Water | ad 100% | ad 100% | ad 100% |
| Titanium Dioxide [29] | 3,00% | 3,00% | 3,00% |
| Talc | 2,00% | 2,00% | 2,00% |
| Iron Oxide [30] | 0,36% | 0,36% | 0,36% |
| Iron Oxide [31] | 0,12% | 0,12% | 0,12% |
| Iron Oxide [32] | 0,08% | 0,08% | 0,08% |
| Isoamyl Cocoate [6] | 4,44% | 4,44% | 4,44% |
| Phenoxyethyl Caprylate [9] | 4,00% | 4,00% | 4,00% |
| Nylon-10/10 [33] | 3,00% | 3,00% | 3,00% |
| Glycerin | 3,00% | 3,00% | 3,00% |
| Xanthan Gum [1] | 0,50% | 0,50% | 0,50% |
| Ethanol | 3,00% | 3,00% | 3,00% |

(fortgesetzt)

| Rezeptur | 14-1 | 14-2 | 14-3 |
|---|---|---|---|
| Methylisothiazolinone, Methylparaben, Ethylparaben; Dipropylene Glycol [3] | 0,80% | 0,80% | 0,80% |
| [27] TEGO Care 450 (Evonik Industries AG)<br>[28] Uvinul A Plus (BASF SE)<br>[29] Hombitan AC 360 (Sachtleben)<br>[30] Unipure Yellow LC 182 (Sensient Technologies)<br>[31] Unipure Red LC 381 (Sensient Technologies)<br>[32] Unipure Black LC 989 (Sensient Technologies)<br>[33] TEGOLON ECO 10-10 (Evonik Industries AG) | | | |

Sonnenschutzlotion SPF 30

| Rezeptur | 15-1 | 15-2 | 15-3 |
|---|---|---|---|
| Emulgator 1 | 3,00% | | |
| Emulgator 4 | | 3,00% | 2,00% |
| Cetearyl Glucoside [5] | | | 0,50% |
| Phenoxyethyl Caprylate [9] | 8,00% | 8,00% | 8,00% |
| Diethylamino Hydroxybenzoyl Hexyl Benzoate [28] | 6,00% | 6,00% | 6,00% |
| Ethylhexyl Methoxycinnamate | 8,00% | 8,00% | 8,00% |
| Stearyl Alcohol | 1,00% | 1,00% | 1,00% |
| Glyceryl Stearate | 1,00% | 1,00% | 1,00% |
| Tocopheryl Acetate | 0,50% | 0,50% | 0,50% |
| Glycerin | 2,00% | 2,00% | 2,00% |
| Demineralized Water | ad 100% | ad 100% | ad 100% |
| Tromethamin | 0,90% | 0,90% | 0,90% |
| Phenylbenzimidazol Sulfonic Acid | 2,00% | 2,00% | 2,00% |
| Acrylates / C10-30 Alkyl Acrylate Crosspolymer [34] | 0,30% | 0,30% | 0,30% |
| Sodium Hydroxide (10 % aq.) | q.s. | q.s. | q.s. |
| Dipropylene Glycol; Methylparaben;Ethylparaben; Aqua;Methylisothiazolinone [3] | 0,80% | 0,80% | 0,80% |
| [34] TEGO® Carbomer 341 ER (Evonik Industries AG) | | | |

Sonnenschutzlotion SPF 30, hoher UVA-Schutz

| Rezeptur | 16-1 | 16-2 | 16-3 |
|---|---|---|---|
| Emulgator 1 | 3,00% | | |
| Emulgator 4 | | 3,50% | 2,50% |
| Sodium Cetearyl Sulfate [16] | | | 0,50% |
| Phenoxyethyl Caprylate [9] | 7,30% | 7,30% | 7,30% |
| Butyl Methoxydibenzoylmethane | 5,00% | 5,00% | 5,00% |
| Diethylhexyl Butamido Triazone [35] | 1,00% | 1,00% | 1,00% |

(fortgesetzt)

| Rezeptur | 16-1 | 16-2 | 16-3 |
|---|---|---|---|
| Ethylhexyl Salicylate | 1,50% | 1,50% | 1,50% |
| Octocrylene | 3,50% | 3,50% | 3,50% |
| Titanium Dioxide; Diethylhexyl Carbonate; Polyglyceryl-6 Polyhydroxystearate [36] | 2,20% | 2,20% | 2,20% |
| Stearyl Alcohol | 1,00% | 1,00% | 1,00% |
| Nylon-10/10 [33] | 0,50% | 0,50% | 0,50% |
| Glyceryl Stearate | 1,00% | 1,00% | 1,00% |
| Tocopheryl Acetate | 0,50% | 0,50% | 0,50% |
| Glycerin | 2,00% | 2,00% | 2,00% |
| Demineralized Water | ad 100% | ad 100% | ad 100% |
| Citric Acid (50% aq.) | 0,10% | 0,10% | 0,10% |
| Bis-Ethylhexyloxyphenol Methoxyphenyl Triazine [8] | 20,00% | 20,00% | 20,00% |
| Acrylates / C10-30 Alkyl Acrylate Crosspolymer [34] | 0,30% | 0,30% | 0,30% |
| Sodium Hydroxide (10% a.q.) | 0,90% | 0,90% | 0,90% |
| Dipropylene Glycol; Methylparaben;Ethylparaben; Aqua;Methylisothiazolinone [4] | 0,80% | 0,80% | 0,80% |
| [35] UVAsorb HEB (3V Sigma) [36] TEGO Sun TDEC 45 (Evonik Industries AG) | | | |

Sonnenschutzlotion SPF 30

| Rezeptur | 17-1 | 17-2 | 17-3 |
|---|---|---|---|
| Emulgator 1 | 3,00% | | 2,00% |
| Emulgator 4 | | 3,00% | |
| Methylglucose Sesquistearate [37] | | | 1,00% |
| Phenoxyethyl Caprylate [9] | 1,50% | 1,50% | 1,50% |
| Octocrylene | 10,00% | 10,00% | 10,00% |
| Butyl Methoxydibenzoylmethane | 3,50% | 3,50% | 3,50% |
| Titanium Dioxide; Diethylhexyl Carbonate; Polyglyceryl-6 Polyhydroxystearate [36] | 14,50% | 14,50% | 14,50% |
| Stearyl Alcohol | 0,20% | 0,20% | 0,20% |
| Glyceryl Stearate | 0,20% | 0,20% | 0,20% |
| Tocopheryl Acetate | 0,50% | 0,50% | 0,50% |
| Glycerin | 2,00% | 2,00% | 2,00% |
| Demineralized Water | ad 100% | ad 100% | ad 100% |
| Acrylates / C10-30 Alkyl Acrylate Crosspolymer [34] | 0,20% | 0,20% | 0,20% |
| Sodium Hydroxide (10% aq.) | 0,60% | 0,60% | 0,60% |
| Dipropylene Glycol; Methylparaben;Ethylparaben; Aqua;Methylisothiazolinone [3] | 0,80% | 0,80% | 0,80% |
| [37] TEGO Care PS (Evonik Industries AG) | | | |

Sonnenschutzlotion SPF 50, hoher UVA-Schutz

| Rezeptur | 18-1 | 18-2 | 18-3 |
|---|---|---|---|
| Emulgator 1 | 3,00% | | 2,50% |
| Emulgator 4 | | 3,00% | |
| Polyglyceryl-3 Methylglucose Distearate [27] | | | 0,50% |
| Phenoxyethyl Caprylate [9] | 2,00% | 2,00% | 2,00% |
| Nylon-10/10 [33] | 0,50% | 0,50% | 0,50% |
| Butyl Methoxydibenzoylmethane | 5,00% | 5,00% | 5,00% |
| Diethylhexyl Butamido Triazone [35] | 1,00% | 1,00% | 1,00% |
| Bis-Ethylhexyloxyphenol Methoxyphenyl Triazine [8] | 3,00% | 3,00% | 3,00% |
| Ethylhexyl Salicylate | 1,00% | 1,00% | 1,00% |
| Octocrylene | 8,00% | 8,00% | 8,00% |
| Stearyl Alcohol | 0,45% | 0,45% | 0,45% |
| Glyceryl Stearate | 0,45% | 0,45% | 0,45% |
| Xanthan Gum [1] | 0,30% | 0,30% | 0,30% |
| Tocopheryl Acetate | 0,50% | 0,50% | 0,50% |
| Hydroxyethylcellulose [14] | 0,50% | 0,50% | 0,50% |
| Demineralized Water | ad 100% | ad 100% | ad 100% |
| Sodium Hydroxide (10% aq., pH-Wert-Einstellung auf 7,5) | q.s. | q.s. | q.s. |
| Disodium Phenyl Dibenzimidazole Tetrasulfonate [38] | 5,00% | 5,00% | 5,00% |
| Methylene Bis-Benzotriazolyl Tetramethylbutylphenol [39] | 8,00% | 8,00% | 8,00% |
| Dipropylene Glycol; Methylparaben;Ethylparaben; Aqua;Methylisothiazolinone [3] | 0,80% | 0,80% | 0,80% |
| [38] Neoheliopan AP (Symrise) <br> [39] Tinosorb M (BASF SE) | | | |

Sonnenschutzlotion SPF 50

| Rezeptur | 19-1 | 19-2 | 19-3 |
|---|---|---|---|
| Emulgator 1 | 3,50% | | |
| Emulgator 4 | | 3,50% | 3,00% |
| Glyceryl Stearate Citrate [7] | | | 1,00% |
| Phenoxyethyl Caprylate [9] | 2,50% | 2,50% | 2,50% |
| Diethylamino Hydroxybenzoyl Hexyl Benzoate | 10,00% | 10,00% | 10,00% |
| Ethylhexyl Methoxycinnamate | 10,00% | 10,00% | 10,00% |
| Stearyl Alcohol | 1,00% | 1,00% | 1,00% |
| Glyceryl Stearate | 1,00% | 1,00% | 1,00% |
| Xanthan Gum [1] | 0,50% | 0,50% | 0,50% |
| Tocopheryl Acetate | 0,50% | 0,50% | 0,50% |
| Glycerin | 2,00% | 2,00% | 2,00% |
| Demineralized Water | ad 100% | ad 100% | ad 100% |

(fortgesetzt)

| Rezeptur | 19-1 | 19-2 | 19-3 |
|---|---|---|---|
| Phenylbenzimidazol Sulfonic Acid | 4,00% | 4,00% | 4,00% |
| Tromethamin | 1,80% | 1,80% | 1,80% |
| Dipropylene Glycol; Methylparaben;Ethylparaben; Aqua;Methylisothiazolinone [3] | 0,80% | 0,80% | 0,80% |

Sonnenschutzlotion SPF 50+

| Rezeptur | 20-1 | 20-2 | 20-3 |
|---|---|---|---|
| Emulgator 1 | 3,00% | | 2,5% |
| Emulgator 4 | | 3,00% | |
| Potassium Cetyl Phosphate | | | 1,0% |
| Phenoxyethyl Caprylate [9] | 3,00% | 3,00% | 3,00% |
| Butyl Methoxydibenzoylmethane | 5,00% | 5,00% | 5,00% |
| Octocrylene | 4,90% | 4,90% | 4,90% |
| Ethylhexyl Methoxycinnamate | 0,10% | 0,10% | 0,10% |
| Bis-Ethylhexyloxyphenol Methoxyphenyl Triazine [8] | 4,70% | 4,70% | 4,70% |
| Diethylhexyl Butamido Triazone [35] | 3,70% | 3,70% | 3,70% |
| Titanium Dioxide; Diethylhexyl Carbonate; Polyglyceryl-6 Polyhydroxystearate [36] | 11,00% | 11,00% | 11,00% |
| Stearyl Alcohol | 0,50% | 0,50% | 0,50% |
| Glyceryl Stearate | 0,50% | 0,50% | 0,50% |
| Xanthan Gum [1] | 0,20% | 0,20% | 0,20% |
| Glycerin | 2,00% | 2,00% | 2,00% |
| Demineralized Water | ad 100% | ad 100% | ad 100% |
| Dipropylene Glycol; Methylparaben;Ethylparaben; Aqua;Methylisothiazolinone [3] | 0,80% | 0,80% | 0,80% |

Lotion für sensitive Haut

| Rezeptur | 21 | 22 | 23 |
|---|---|---|---|
| Emulgator 1 | 3,0% | 3,0% | 3,0% |
| Glyceryl Stearate | 0,5% | 1,0% | 0,5% |
| Cetearyl Alcohol | 1,0% | 1,0% | 1,0% |
| Butyrospermum Parkii (Shea) Butter | 3,0% | 3,0% | 3,0% |
| Caprylic/Capric Triglyceride | 5,0% | 5,0% | 5,0% |
| Isopropyl Palmitate | 5,0% | 5,0% | 5,0% |
| Xanthan Gum [1] | 0,2% | 0,2% | 0,2% |
| Glycerin | 5,0% | 5,0% | 5,0% |
| Urea | 10,0% | 15,0% | 20,0% |
| Demineralized Water | ad 100% | ad 100% | ad 100% |

(fortgesetzt)

| Rezeptur | 21 | 22 | 23 |
|---|---|---|---|
| Phenoxyethanol, Ethylhexylglycerin [40] | 0,7% | | |
| Sodium Hydroxide (10% aq.)(pH-Wert-Einstellung auf 5,0) | | q.s. | |
| Sodium Benzoate, Potassium Sorbate [41] | | 1,2% | |
| Methylisothiazolinone, Methylparaben, Ethylparaben; Dipropylene Glycol [3] | | | 0,8% |
| [40] Euxyl PE 9010 (Schülke & Mayr GmbH)  [41] Euxyl K 712 (Schülke & Mayr GmbH) | | | |

Pflegende Lotion für trockene Haut 1

| Rezeptur | 24-1 | 24-2 | 24-3 | 25-1 | 25-2 |
|---|---|---|---|---|---|
| Emulgator 1 | 3,0% | | | 3,0% | |
| Emulgator 4 | | 3,0% | 2,5% | | 3,0% |
| Sodium Stearoyl Glutamate [12] | | | 1,0% | | |
| Glyceryl Stearate | 1,0% | 1,0% | 1,0% | 1,5% | 1,5% |
| Cetearyl Alcohol | 1,0% | 1,0% | 1,0% | 1,5% | 1,5% |
| Cetyl Ricinoleate | 2,0% | 2,0% | 2,0% | 2,0% | 2,0% |
| Oleyl Erucate [22] | 5,0% | 5,0% | 5,0% | 5,0% | 5,0% |
| Isoamyl Cocoate [6] | 8,0% | 8,0% | 8,0% | 8,0% | 8,0% |
| Decyl Cocoate [42] | 3,0% | 3,0% | 3,0% | 3,0% | 3,0% |
| Xanthan Gum [1] | 0,2% | 0,2% | 0,2% | 0,2% | 0,2% |
| Glycerin | 5,0% | 5,0% | 5,0% | 5,0% | 5,0% |
| Urea | 10,0% | 10,0% | 10,0% | 15,0% | 15,0% |
| Demineralized Water | ad 100% | ad 100% | ad 100% | ad 100% | ad 100% |
| Methylisothiazolinone, Methylparaben, Ethylparaben; Dipropylene Glycol [3] | 0,8% | 0,8% | 0,8% | 0,8% | 0,8% |
| [42] TEGOSOFT DC (Evonik Industries AG) | | | | | |

Pflegende Lotion für trockene Haut 2

| Rezeptur | 25-3 | 26-1 | 26-2 | 26-3 |
|---|---|---|---|---|
| Emulgator 1 | | 3,0% | | 3,0% |
| Emulgator 4 | 2,5% | | 3,0% | |
| Polyglyceryl-6 Distearate | 0,5% | | | |
| Stearic Acid | | | | 0,5% |
| Glyceryl Stearate | 1,5% | 1,5% | 1,5% | 1,5% |
| Cetearyl Alcohol | 1,5% | 1,5% | 1,5% | 1,5% |
| Cetyl Ricinoleate | 2,0% | 2,0% | 2,0% | 2,0% |
| Oleyl Erucate [22] | 5,0% | 5,0% | 5,0% | 5,0% |
| Isoamyl Cocoate [6] | 8,0% | 8,0% | 8,0% | 8,0% |
| Decyl Cocoate [42] | 3,0% | 3,0% | 3,0% | 3,0% |

(fortgesetzt)

| Rezeptur | 25-3 | 26-1 | 26-2 | 26-3 |
|---|---|---|---|---|
| Xanthan Gum [1] | 0,2% | 0,2% | 0,2% | 0,2% |
| Glycerin | 5,0% | 5,0% | 5,0% | 5,0% |
| Urea | 15,0% | 20,0% | 20,0% | 20,0% |
| Demineralized Water | ad 100% | ad 100% | ad 100% | ad 100% |
| Methylisothiazolinone, Methylparaben, Ethylparaben; Dipropylene Glycol [3] | 0,8% | 0,8% | 0,8% | 0,8% |
| [42] TEGOSOFT DC (Evonik Industries AG) | | | | |

### Konservierungsmittelfreie Lotionen 1

| Rezeptur | 27-1 | 27-2 | 27-3 | 28-1 | 28-2 |
|---|---|---|---|---|---|
| Emulgator 1 | 3,0% | | 2,0% | 3,0% | |
| Emulgator 4 | | 3,0% | | | 3,0% |
| Glyceryl Stearate Citrate [7] | | | 1,0% | | |
| Glyceryl Stearate SE | | | | | |
| Polyglyceryl-3 Dicitrate/Stearate [21] | | | | | |
| Glyceryl Stearate | 0,2% | 0,2% | 0,2% | 0,5% | 0,5% |
| Stearyl Alcohol | 0,2% | 0,2% | 0,2% | 0,5% | 0,5% |
| Prunus Amygdalus Dulcis (Sweet Almond) Oil | 10,0% | 10,0% | 10,0% | 10,0% | 10,0% |
| Isoamyl Cocoate [6] | 6,6% | 6,6% | 6,6% | 6,0% | 6,0% |
| Glycerin | 4,0% | 4,0% | 4,0% | 4,0% | 4,0% |
| Demineralized Water | ad 100,0% | ad 100,0% | ad 100,0% | ad 100,0% | ad 100,0% |
| Caprylyl Glycol, Glycerin, Glyceryl Caprylate, Phenylpropanol [43] | 1,0% | 1,0% | 1,0% | | |
| Methylpropanediol, Caprylyl Glycol, Phenylpropanol [44] | | | | 4,0% | 4,0% |
| Xanthan Gum [1] | 0,5% | 0,5% | 0,5% | 0,5% | 0,5% |

[43] Dermosoft LP (Dr. Straetmans Chemische Produkte GmbH)
[44] Dermosoft OMP (Dr. Straetmans Chemische Produkte GmbH)
[45] Dermosoft Octiol (Dr. Straetmans Chemische Produkte GmbH)
[46] 3,0g Natrium Hydroxid in 56,5g demineralisiertem Wasser lösen, 30,0g Glycerin und 10,0g Dermosoft Octiol (Dr. Straetmans Chemische Produkte GmbH) zugeben, rühren bis Lösung klar ist.

### Konservierungsmittelfreie Lotionen 2

| Rezeptur | 28-3 | 29-1 | 29-2 | 29-3 |
|---|---|---|---|---|
| Emulgator 1 | | 3,0% | | 1,5% |
| Emulgator 4 | 3,0% | | 3,0% | |
| Glyceryl Stearate SE | 0,5% | | | |
| Polyglyceryl-3 Dicitrate/Stearate [21] | | | | 1,5% |
| Glyceryl Stearate | 0,5% | 0,5% | 0,5% | 0,5% |

(fortgesetzt)

| Rezeptur | 28-3 | 29-1 | 29-2 | 29-3 |
|---|---|---|---|---|
| Stearyl Alcohol | 0,5% | 0,5% | 0,5% | 0,5% |
| Prunus Amygdalus Dulcis (Sweet Almond) Oil | 10,0% | 10,0% | 10,0% | 10,0% |
| Isoamyl Cocoate [6] | 6,0% | 6,0% | 6,0% | 6,0% |
| Glycerin | 4,0% | 4,0% | 4,0% | 4,0% |
| Demineralized Water | ad 100,0% | ad 100,0% | ad 100,0% | ad 100,0% |
| Methylpropanediol, Caprylyl Glycol, Phenylpropanol [44] | 4,0% | | | |
| Caprylyl Glycol [45] | | 0,4% | 0,4% | 0,4% |
| p-Anisic Acid-Lösung 10%ig [46] | | 2,0% | 2,0% | 2,0% |
| Citric Acid (10% aq.) (pH-Wert-Einstellung auf 6,0) | | q.s. | q.s. | q.s. |
| Xanthan Gum [1] | 0,5% | 0,5% | 0,5% | 0,5% |

**Patentansprüche**

1. Polyglycerinester,
der nach seiner vollständiger Hydrolyse im Mittel (Zahlenmittel) pro Mol Polyglycerinester von 0,51 bis 2,20 Mol, bevorzugt von 0,73 bis 1,85 Mol, besonders bevorzugt von 0,85 bis 1,50 Mol, mindestens einer Carbonsäure mit 8 bis 24 Kohlenstoffatomen freisetzt,
**dadurch gekennzeichnet, dass** nach vollständiger Hydrolyse des Polyglycerinesters ein Polyglycerin erhalten wird, in dem das Massenverhältnis von Glycerin zu Diglycerin größer 1, bevorzugt größer 1,2, besonders bevorzugt größer 1,5 ist, und
das einen mittleren Polymerisationsgrad von 3 bis 20, bevorzugt von 3,5 bis 15 besonders bevorzugt von 4,5 bis 10 aufweist.

2. Polyglycerinester gemäß Anspruch 1, **dadurch gekennzeichnet, dass** nach seiner vollständigen Hydrolyse im Mittel (Zahlenmittel) pro Mol Polyglycerinester von 0,01 bis 0,20 Mol, bevorzugt von 0,03 bis 0,15 Mol, besonders bevorzugt von 0,05 bis 0,10 Mol, mindestens einer ersten Carbonsäure mit 20 bis 24 Kohlenstoffatomen und von 0,5 bis 2,0 Mol, bevorzugt von 0,7 bis 1,7 Mol, besonders bevorzugt von 0,8 bis 1,4 Mol, mindestens einer zweiten Carbonsäure mit 8 bis 18, bevorzugt mit 14 bis 18, besonders bevorzugt von 16 bis 18 Kohlenstoffatomen, freigesetzt werden.

3. Polyglycerinester gemäß mindestens einem der vorherigen Ansprüche, **dadurch gekennzeichnet, dass** das Molverhältnis der nach vollständiger Hydrolyse des Polyglycerinesters erhaltenen ersten Carbonsäure zur zweiten Carbonsäure zwischen 1 zu 50 bis 1 zu 7, bevorzugt zwischen 1 zu 28 bis 1 zu 10, besonders bevorzugt zwischen 1 zu 19 und 1 zu 13, liegt.

4. Polyglycerinester gemäß mindestens einem der vorherigen Ansprüche, **dadurch gekennzeichnet, dass** die erste und zweite Carbonsäure ausgewählt ist aus linearen, gesättigten, unsubstituierten Carbonsäuren.

5. Polyglycerinester gemäß mindestens einem der vorherigen Ansprüche, **dadurch gekennzeichnet, dass** die erste Carbonsäure Behensäure und die zweite Carbonsäure eine Mischung von Stearinsäure und Palmitinsäure ist.

6. Verfahren zur Herstellung eines Polyglycerinesters umfassend die Verfahrensschritte

A) Bereitstellen eines Polyglycerins, in dem das Massenverhältnis von Glycerin zu Diglycerin größer 1, bevorzugt größer 1,2, besonders bevorzugt größer 1,5 ist und aufweisend einen mittleren Polymerisationsgrad von 3 bis 20, bevorzugt von 3,5 bis 15 besonders bevorzugt von 4,5 bis 10,
B) Verestern mindestens eines Teils des Polyglycerins mit mindestens einer Carbonsäure mit 8 bis 24 Kohlenstoffatomen,

wobei das molare Verhältnis der in Verfahrensschritt B) eingesetzten Carbonsäure zu in Verfahrensschritt A) eingesetztem Polyglycerin von 0,51 zu 1 bis 2,20 zu 1, bevorzugt von 0,73 zu 1 bis 1,85 zu 1, besonders bevorzugt von 0,85 zu 1 bis 1,5 zu 1 beträgt.

7. Verfahren gemäß Anspruch 6, **dadurch gekennzeichnet, dass** in Verfahrensschritt B) mindestens eine erste Carbonsäure mit 20 bis 24 Kohlenstoffatomen und mindestens eine zweite Carbonsäure mit 8 bis 18 Kohlenstoffatomen eingesetzt wird,
wobei das molare Verhältnis der in Verfahrensschritt B) eingesetzten ersten Carbonsäure zu in Verfahrensschritt A) eingesetztem Polyglycerin von 0,01 zu 1 bis 0,20 zu 1, bevorzugt von 0,03 zu 1 bis 0,15 zu 1, besonders bevorzugt von 0,05 zu 1 bis 0,10 zu 1, und das molare Verhältnis der in Verfahrensschritt B) eingesetzten zweiten Carbonsäure zu in Verfahrensschritt A) eingesetztem Polyglycerin von 0,5 zu 1 bis 2,0 zu 1, bevorzugt von 0,7 zu 1 bis 1,7 zu 1, besonders bevorzugt von 0,8 zu 1 bis 1,4 zu 1, beträgt.

8. Verfahren gemäß Anspruch 7, **dadurch gekennzeichnet, dass** in Verfahrensschritt B) als die erste Carbonsäure Behensäure und als die zweite Carbonsäure eine Mischung von Stearinsäure und Palmitinsäure eingesetzt werden.

9. Verwendung des Polyglycerinesters gemäß mindestens einem der Ansprüche 1 bis 5 oder des Polyglycerinesters erhältlich nach einem Verfahren gemäß mindestens einem der Ansprüche 6 bis 8 als Emulgator.

10. Verwendung des Polyglycerinesters gemäß mindestens einem der Ansprüche 1 bis 5 oder des Polyglycerinesters erhältlich nach einem Verfahren gemäß mindestens einem der Ansprüche 6 bis 8 zur Herstellung von kosmetischen oder pharmazeutischen Formulierungen.

11. Emulgator enthaltend Polyglycerinester gemäß mindestens einem der Ansprüche 1 bis 5 oder des Polyglycerinesters erhältlich nach einem Verfahren gemäß mindestens einem der Ansprüche 6 bis 8.

12. Kosmetische oder pharmazeutische Formulierung enthaltend Polyglycerinester gemäß mindestens einem der Ansprüche 1 bis 5 oder Polyglycerinester erhältlich nach einem Verfahren gemäß mindestens einem der Ansprüche 6 bis 8.

13. Kosmetische oder pharmazeutische Formulierung gemäß Anspruch 12, **dadurch gekennzeichnet, dass** der Polyglycerinester in Mengen von 0,1 bis 10 Gew.-% enthalten ist.

14. Kosmetische oder pharmazeutische Formulierung gemäß Anspruch 12 oder 13, zusätzlich enthaltend eine Substanz ausgewählt aus der Gruppe der Substanzen der UV-Lichtschutzfilter, insbesondere der wasserlöslichen UV-Lichtschutzfilter.

15. Kosmetische oder pharmazeutische Formulierung gemäß mindestens einem der Ansprüche 12 bis 14, zusätzlich enthaltend eine Substanz ausgewählt aus der Gruppe der Substanzen der Insektrepellentien.

**Claims**

1. Polyglycerol ester,
which, after its complete hydrolysis, releases on average (number-average) per mole of polyglycerol ester, from 0.51 to 2.20 mol, preferably from 0.73 to 1.85 mol, particularly preferably from 0.85 to 1.50 mol, of at least one carboxylic acid having 8 to 24 carbon atoms,
**characterized in that**, after complete hydrolysis of the polycerol ester, a polygerol is obtained in which the mass ratio of glycerol to diglycerol is greater than 1, preferably greater than 1.2, particularly preferably greater than 1.5, and which has an average degree of polymerization of from 3 to 20, preferably from 3.5 to 15, particularly preferably from 4.5 to 10.

2. Polyglycerol ester according to Claim 1, **characterized in that**, after its complete hydrolysis, on average (number-average) per mole of polyglycerol ester, from 0.01 to 0.20 mol, preferably from 0.03 to 0.15 mol, particularly preferably from 0.05 to 0.10 mol, of at least one first carboxylic acid having 20 to 24 carbon atoms and from 0.5 to 2.0 mol, preferably from 0.7 to 1.7 mol, particularly preferably from 0.8 to 1.4 mol, of at least one second carboxylic acid having 8 to 18, preferably having 14 to 18, particularly preferably from 16 to 18, carbon atoms, are released.

3. Polyglycerol ester according to at least one of the preceding claims, **characterized in that** the molar ratio of the first carboxylic acid obtained after complete hydrolysis of the polyglycerol ester relative to the second carboxylic acid is between 1:50 to 1:7, preferably between 1:28 and 1:10, particularly preferably between 1:19 and 1:13.

4. Polyglycerol ester according to at least one of the preceding claims, **characterized in that** the first and second carboxylic acid is selected from linear, saturated, unsubstituted carboxylic acids.

5. Polyglycerol ester according to at least one of the preceding claims, **characterized in that** the first carboxylic acid is behenic acid and the second carboxylic acid is a mixture of stearic acid and palmitic acid.

6. Process for producing a polyglycerol ester comprising the process steps

   A) provision of a polyglycerol in which the mass ratio of glycerol to diglycerol is greater than 1, preferably greater than 1.2, particularly preferably greater than 1.5 and having an average degree of polymerization of from 3 to 20, preferably from 3.5 to 15, particularly preferably of from 4.5 to 10,
   B) esterification of at least some of the polyglycerol with at least one carboxylic acid having 8 to 24 carbon atoms,

   where the molar ratio of the carboxylic acid used in process step B) to polyglycerol used in process step A) is from 0.51:1 to 2.20:1, preferably from 0.73:1 to 1.85:1, particularly preferably from 0.85:1 to 1.5:1.

7. Process according to Claim 6, **characterized in that** in process step B) at least one first carboxylic acid having 20 to 24 carbon atoms and at least one second carboxylic acid having 8 to 18 carbon atoms is used, where the molar ratio of the first carboxylic acid used in process step B) to polyglycerol used in process step A) is from 0.01:1 to 0.20:1, preferably from 0.03:1 to 0.15:1, particularly preferably from 0.05:1 to 0.10:1, and the molar ratio of the second carboxylic acid used in process step B) to polyglycerol used in process step A) is from 0.5:1 to 2.0:1, preferably from 0.7:1 to 1.7:1, particularly preferably from 0.8:1 to 1.4:1.

8. Process according to Claim 7, **characterized in that**, in process step B), the first carboxylic acid used is behenic acid and the second carboxylic acid used is a mixture of stearic acid and palmitic acid.

9. Use of the polyglycerol ester according to at least one of Claims 1 to 5 or of the polyglycerol ester obtainable by a process according to at least one of Claims 6 to 8 as emulsifier.

10. Use of the polyglycerol ester according to at least one of Claims 1 to 5 or of the polyglycerol ester obtainable by a process according to at least one of Claims 6 to 8 for producing cosmetic or pharmaceutical formulations.

11. Emulsifier comprising polyglycerol ester according to at least one of Claims 1 to 5 or of the polyglycerol ester obtainable by a process according to at least one of Claims 6 to 8.

12. Cosmetic or pharmaceutical formulation comprising polyglycerol ester according to at least one of Claims 1 to 5 or polyglycerol ester obtainable by a process according to at least one of Claims 6 to 8.

13. Cosmetic or pharmaceutical formulation according to Claim 12, **characterized in that** the polyglycerol ester is present in amounts of from 0.1 to 10% by weight.

14. Cosmetic or pharmaceutical formulation according to Claim 12 or 13, additionally comprising a substance selected from the group of the substances of the UV light protection filters, in particular of the water-soluble UV light protection filters.

15. Cosmetic or pharmaceutical formulation according to at least one of Claims 12 to 14, additionally comprising a substance selected from the group of the substances of the insect repellents.

**Revendications**

1. Ester de polyglycérine,
   qui libère après son hydrolyse totale en moyenne (moyenne en nombre), par mole d'ester de polyglycérine, de 0,51 à 2,20 moles, de préférence de 0,73 à 1,85 mole, de manière particulièrement préférée de 0,85 à 1,50 mole, d'au moins un acide carboxylique de 8 à 24 atomes de carbone,

**caractérisé en ce qu'**après l'hydrolyse totale de l'ester de polyglycérine, une polyglycérine est obtenue, dans laquelle le rapport en masse entre la glycérine et la diglycérine est supérieur à 1, de préférence supérieur à 1,2, de manière particulièrement préférée supérieur à 1,5, et

qui présente un degré de polymérisation moyen de 3 à 20, de préférence de 3,5 à 15, de manière particulièrement préférée de 4,5 à 10.

2. Ester de polyglycérine selon la revendication 1, **caractérisé en ce qu'**après son hydrolyse totale, en moyenne (moyenne en nombre), par mole d'ester de polyglycérine, de 0,01 à 0,20 mole, de préférence de 0,03 à 0,15 mole, de manière particulièrement préférée de 0,05 à 0,10 mole, d'au moins un premier acide carboxylique de 20 à 24 atomes de carbone, et

de 0,5 à 2,0 moles, de préférence de 0,7 à 1,7 mole, de manière particulièrement préférée de 0,8 à 1,4 mole, d'au moins un deuxième acide carboxylique de 8 à 18, de préférence de 14 à 18, de manière particulièrement préférée de 16 à 18 atomes de carbone, sont libérés.

3. Ester de polyglycérine selon au moins l'une quelconque des revendications précédentes, **caractérisé en ce que** le rapport molaire entre le premier acide carboxylique et le deuxième acide carboxylique obtenus après l'hydrolyse totale de l'ester de polyglycérine est compris entre 1 sur 50 et 1 sur 7, de préférence entre 1 sur 28 et 1 sur 10, de manière particulièrement préférée entre 1 sur 19 et 1 sur 13.

4. Ester de polyglycérine selon au moins l'une quelconque des revendications précédentes, **caractérisé en ce que** le premier et le deuxième acide carboxylique sont choisis parmi les acides carboxyliques linéaires, saturés, non substitués.

5. Ester de polyglycérine selon au moins l'une quelconque des revendications précédentes, **caractérisé en ce que** le premier acide carboxylique est l'acide béhénique et le deuxième acide carboxylique est un mélange d'acide stéarique et d'acide palmitique.

6. Procédé de fabrication d'un ester de polyglycérine comprenant les étapes de procédé suivantes :

A) la préparation d'une polyglycérine dans laquelle le rapport en masse entre la glycérine et la diglycérine est supérieur à 1, de préférence supérieur à 1,2, de manière particulièrement préférée supérieur à 1,5, et présentant un degré de polymérisation moyen de 3 à 20, de préférence de 3,5 à 15, de manière particulièrement préférée de 4,5 à 10,

B) l'estérification d'au moins une partie de la polyglycérine avec au moins un acide carboxylique de 8 à 24 atomes de carbone,

le rapport molaire entre l'acide carboxylique utilisé à l'étape de procédé B) et la polyglycérine utilisée à l'étape de procédé A) étant de 0,51 sur 1 à 2,20 sur 1, de préférence de 0,73 sur 1 à 1,85 sur 1, de manière particulièrement préférée de 0,85 sur 1 à 1,5 sur 1.

7. Procédé selon la revendication 6, **caractérisé en ce qu'**à l'étape de procédé B), au moins un premier acide carboxylique de 20 à 24 atomes de carbone et au moins un deuxième acide carboxylique de 8 à 18 atomes de carbone sont utilisés,

le rapport molaire entre le premier acide carboxylique utilisé à l'étape de procédé B) et la polyglycérine utilisée à l'étape de procédé A) étant de 0,01 sur 1 à 0,20 sur 1, de préférence de 0,03 sur 1 à 0,15 sur 1, de manière particulièrement préférée de 0,05 sur 1 à 0,10 sur 1, et le rapport molaire entre le deuxième acide carboxylique utilisé à l'étape de procédé B) et la polyglycérine utilisée à l'étape de procédé A) étant de 0,5 sur 1 à 2,0 sur 1, de préférence de 0,7 sur 1 à 1,7 sur 1, de manière particulièrement préférée de 0,8 sur 1 à 1,4 sur 1.

8. Procédé selon la revendication 7, **caractérisé en ce qu'**à l'étape de procédé B), l'acide béhénique est utilisé en tant que premier acide carboxylique, et un mélange d'acide stéarique et d'acide palmitique est utilisé en tant que deuxième acide carboxylique.

9. Utilisation de l'ester de polyglycérine selon au moins l'une quelconque des revendications 1 à 5 ou de l'ester de polyglycérine pouvant être obtenu par un procédé selon au moins l'une quelconque des revendications 6 à 8 en tant qu'émulsifiant.

**10.** Utilisation de l'ester de polyglycérine selon au moins l'une quelconque des revendications 1 à 5 ou de l'ester de polyglycérine pouvant être obtenu par un procédé selon au moins l'une quelconque des revendications 6 à 8 pour la fabrication de formulations cosmétiques ou pharmaceutiques.

**11.** Émulsifiant contenant un ester de polyglycérine selon au moins l'une quelconque des revendications 1 à 5 ou un ester de polyglycérine pouvant être obtenu par un procédé selon au moins l'une quelconque des revendications 6 à 8.

**12.** Formulation cosmétique ou pharmaceutique contenant un ester de polyglycérine selon au moins l'une quelconque des revendications 1 à 5 ou un ester de polyglycérine pouvant être obtenu par un procédé selon au moins l'une quelconque des revendications 6 à 8.

**13.** Formulation cosmétique ou pharmaceutique selon la revendication 12, **caractérisée en ce que** l'ester de polyglycérine est contenu en quantités de 0,1 à 10 % en poids.

**14.** Formulation cosmétique ou pharmaceutique selon la revendication 12 ou 13, contenant en outre une substance choisie dans le groupe de substances des filtres de protection contre la lumière UV, notamment des filtres de protection contre la lumière UV solubles dans l'eau.

**15.** Formulation cosmétique ou pharmaceutique selon au moins l'une quelconque des revendications 12 à 14, contenant en outre une substance choisie dans le groupe de substances des insectifuges.

Abbildung 1

Abbildung 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2003104852 B
- JP 2010178723 B
- JP 2006055029 B
- WO 2008103289 A
- EP 1623694 A
- WO 2011098311 A
- DE 102008001788

**In der Beschreibung aufgeführte Nicht-Patentliteratur**

- **K. SCHRADER.** Grundlagen und Rezepturen der Kosmetika. Hüthig Buch Verlag Heidelberg, 329-341
- **P. FINKEL.** *SÖFW-Journal,* 1996, vol. 122, 543